# EUROPÄISCHE PATENTSCHRIFT

(11) **EP 2 364 689 B1**
(45) Veröffentlichungstag und Bekanntmachung des Hinweises auf die Patenterteilung: **11.12.2019**
(21) Anmeldenummer: 11157363.0
(22) Anmeldetag: 06.10.2009
(51) Int. Cl.: A61K 8/34, A61K 8/42, A61Q 11/00, A23G 4/06

(54) **Mentholhaltige Zahnputz-Zusammensetzung mit reduzierter Bitterwahrnehmung**
Teeth cleaning compound containing menthol with reduced bitter sensation
Composition de nettoyage des dents contenant du menthol dotée d'une admission de l'amertume réduite

(43) Veröffentlichungstag der Anmeldung: 14.09.2011
(62) Teilanmeldung aus: 09172357.7
(73) Patentinhaber: Symrise AG, 37603 Holzminden (DE)
(72) Erfinder: Oertling, Heiko, Dr., 37603 Holzminden (DE); Loges, Hubert, 37671 Höxter (DE); Machinek, Arnold, 37603 Holzminden (DE); Simchen, Ulrike, 37603 Holzminden (DE); Surburg, Horst, Dr., 37603 Holzminden (DE)
(74) Vertreter: Eisenführ Speiser

(56) Entgegenhaltungen:
- WO-A1-96/17524
- GB-A- 1 457 671
- US-A- 5 695 746
- US-A1- 2009 148 938

## Beschreibung

Die vorliegende Erfindung betrifft die Verwendung einer Aromastoff-Komposition mit den Komponenten
a) Menthol,
b) eine die Bitterkeit des Menthol maskierende Menge an Menthancarbonsäure-N-(4-methoxyphenyl)-amid (WS-12) sowie
c) ein, zwei oder mehr weitere Aromastoffe, wobei der, mehrere oder sämtliche der weiteren Aromastoffe einen log-K0w-Wert im Bereich von 1,5 bis 5 besitzen,
zur Maskierung unangenehmer Geschmackseindrücke von Triclosan, Zinkcitrat, -sulfat, Poly- und Pyrophosphaten, Bicarbonaten (z.B. Natriumbicarbonat), Strontium- und Kaliumsalzen (z.B. Kaliumcitrat, Kaliumnitrat, Kaliumchlorid, Strontiumchlorid), Zinnpyrophosphat, -chlorid, Aluminiumlactat, Wasserstoffperoxid, Fluoriden, Vitaminen, Cetylpyridiniumchlorid und Emulgatoren, wie z.B. Natriumlaurylsulfat, Natriumlaurylsarkosinat und Cocamidopropylbetain sowie Süßstoffen wie z.B. Aspartam, Saccharin, Acesulfam-K, Sorbit, Xylit, Cyclamaten (z. B. Natriumcyclamat), Sucralose, Alitam, Neotam, Thaumatin, von Neohesperidin Dihydrochalcon, Maltit, Lactit oder Kaugummi-Massen (GumBasen) oder deren Mischungen, wobei die unangenehmen Geschmackseindrücke bittere oder metallische Geschmackseindrücke sind

Einzelheiten der vorliegenden Erfindung sowie weitere Aspekte der Erfindung, insbesondere besonders bevorzugte Ausgestaltungen, ergeben sich aus der folgenden Beschreibung, den Beispielen sowie den beigefügten Patentansprüchen.

Marktübliche Zahnputz-Zusammensetzungen zur Anwendung mit einer Zahnbürste, insbesondere Zahnpasten, Zahncremes, Zahnputzgele und Zahnpulver, enthalten heutzutage regelmäßig eine größere Anzahl von Wirkstoffen, z. B. mit Wirkung gegen Karies, Zahnbelagbildung, Zahnfleischentzündung, Zahnhalsempfindlichkeit, Zahnsteinbildung, schlechten Atem usw.. Diese Wirkstoffe bringen häufig in verstärktem Maße unangenehme Geschmackseindrücke mit sich, so dass die besagten Zahnputz-Zusammensetzungen üblicherweise in erhöhtem Maße aromatisiert sind. So führen Stoffe wie Triclosan, Kalium- oder Zinkcitrat, Zinnchlorid, Zinnfluorid, Aminfluorid, oder Cetylpyridiniumchlorid zu salzigen, metallischen, beißenden, bitteren, adstringierenden, betäubenden, brennenden, seifigen oder muffigen Geschmacksnoten.

Um die als äußerst negativ empfundenen Geschmacksnoten der in Zahnputz-Zusammensetzungen eingesetzten Wirkstoffe zu überdecken oder zu maskieren (d. h. zu reduzieren oder zu vermindern) und um gleichzeitig dem Verbraucher das Gefühl einer angenehmen Frische zu vermitteln, wurde in den vergangenen Jahren der eingesetzte Anteil an Aroma in einer Zahnputz-Zusammensetzung von früher ca. 1 Gew.-% auf heutzutage ca. 1,2 bis 1,3 Gew.-%, in Einzelfällen sogar 1,5 Gew.-% oder mehr erhöht. Die Gew.-%-Angaben beziehen sich dabei auf die fertige Zahnputz-Zusammensetzung. Innerhalb der Aroma-Komponente einer Zahnputz-Zusammensetzung hat sich der Gesamtanteil des eingesetzten Menthols in der Praxis ebenso erhöht wie der Anteil an (linear) als Einzelkomponente zugesetztem Menthol. Früher lag der Gesamtanteil von Menthol häufig im Bereich von 10 bis 20 Gew.-%, bezogen auf die Aromastoff-Komposition innerhalb einer üblichen Zahnputz-Zusammensetzung, heutzutage sind eher Gesamtmengen an Menthol im Bereich von ca. 40 bis 50 Gew.-% üblich, bezogen auf das Gesamtgewicht der Aromastoff-Komposition. Die Gesamtmenge an Menthol ist dabei die Summe an (a) Menthol, welches in Form einer mentholhaltigen Formulierung dem Aroma zugesetzt wurde sowie (b) Menthol, welches (linear) als Einzelkomponente zugesetzt wurde. In den letzten Jahren hat sich somit zusammenfassend der Anteil an Menthol in jeweils marktüblichen Zahnputz-Zusammensetzungen um ca. den Faktor 4 erhöht.

Der über die Jahre immer weiter erhöhte Anteil von Menthol an Zahnputz-Zusammensetzungen bringt neben einer Reihe von Vorteilen auch wesentliche Nachteile mit sich. Zwar überdeckt der erhöhte Anteil von Menthol aufgrund des erhöhten Frische- und Kälteempfindens recht wirksam die vorstehend genannten negativen Geschmacksnoten der in Zahnputz-Zusammensetzungen eingesetzten Wirkstoffe; gleichzeitig bringt Menthol in den nunmehr eingesetzten höheren Mengen aber auch negative geschmackliche Eigenschaften mit sich und wird insbesondere in höheren Konzentrationen häufig als scharf und bitter empfunden. Es besteht daher ein Bedarf an einer Maskierung der vom Menthol verursachten Geschmacksnoten in Zahnputz-Zusammensetzungen.

In der Praxis wird in vielen Fällen Anethol in Zahnputz-Zusammensetzungen eingesetzt, welches aufgrund seiner geschmacklichen Süße in der Lage ist, die Schärfe und Bitterkeit des Menthols zu reduzieren. Anethol besitzt jedoch wiederum selbst einen starken Anis-Geschmack, der von vielen Verbrauchern abgelehnt wird. Aufgrund dieses starken und charakteristischen Eigengeschmacks limitiert die Anwesenheit von Anethol in einer Zahnputz-Zusammensetzung überdies die Verwendung weiterer Aromen mit anderen Geschmacksrichtungen, da sich der Eigengeschmack von Anethol nur schwer mit anderen Geschmacksrichtungen zu einer harmonischen Einheit kombinieren lässt, insbesondere bei Einsatz höherer Konzentrationen. Dies erscheint insbesondere insoweit problematisch, weil es eine zunehmende Nachfrage nach neuen Geschmackseindrücken von Zahnputz-Zusammensetzungen gibt.

Es war Aufgabe der vorliegenden Erfindung, neue, vorteilhafte Aromastoff-Kompositionen anzugeben, welche ein Frische- und Kälteempfinden vermitteln, ohne in nennenswertem oder störendem Umfang eine scharfe oder bittere Geschmacksnote zu vermitteln.

Aufgrund weiterführender Überlegungen zur zukünftigen Gestaltung von Zahnputz-Zusammensetzungen sollte dabei vorzugsweise auf den Einsatz von Menthol in anzugebenden Zahnputz-Zusammensetzungen nicht vollständig verzichtet werden. Eine spezielle Aufgabenstellung im Rahmen der vorliegenden Erfindung war es daher, eine Aromastoff-Komposition anzugeben, welche Menthol enthält, wobei aufgrund anzugebender Maßnahmen das Menthol zwar das gewünschte Frische- und Kälteempfinden vermittelt, nicht jedoch den unerwünschten Geschmack von Schärfe oder Bitterkeit.

Weitere (Teil-) Aufgaben im Rahmen der Erfindung ergeben sich aus den nachfolgenden Ausführungen, insbesondere bei Betrachtung der dort angegebenen Vorteile im Zusammenhang mit hierin beschriebenen Zahnputz-Zusammensetzungen.

Betrachtet man die obigen Ausführungen zu den negativen Aspekten der in Zahnputz-Zusammensetzungen üblicherweise eingesetzten Wirkstoffe (negative Geschmacksnoten), des zur Maskierung dieser negativen Geschmacksnoten eingesetzten Menthols (in größerer Konzentration eigene negative Geschmacksnoten) und schließlich des zur Maskierung der negativen Geschmacksnoten des Menthols eingesetzten Anethols (starker Eigengeschmack mit Limitierung der Variationsmöglichkeiten bei Einsatz weiterer Aromastoffe) so ist festzustellen, dass in üblichen Zahnputz-Zusammensetzungen bereits ein komplexes Wechselspiel geschmacklicher Abhängigkeiten besteht. Dies erklärt, warum im Markt bislang keine überzeugenden Alternativen zu den vorstehend geschilderten Zahnputz-Zusammensetzungen anzutreffen sind.

Die vorstehend genannte primäre Aufgabe wird erfindungsgemäß gelöst durch die Verwendung einer Aromastoff-Komposition mit den Komponenten
a) Menthol,
b) eine die Bitterkeit des Menthol maskierende Menge an Menthancarbonsäure-N-(4-methoxyphenyl)-amid (WS-12) sowie
c) ein, zwei oder mehr weitere Aromastoffe, wobei der, mehrere oder sämtliche der weiteren Aromastoffe einen log-K0w-Wert im Bereich von 1,5 bis 5 besitzen,
zur Maskierung unangenehmer Geschmackseindrücke von Triclosan, Zinkcitrat, -sulfat, Poly- und Pyrophosphaten, Bicarbonaten (z.B. Natriumbicarbonat), Strontium- und Kaliumsalzen (z.B. Kaliumcitrat, Kaliumnitrat, Kaliumchlorid, Strontiumchlorid), Zinnpyrophosphat, -chlorid, Aluminiumlactat, Wasserstoffperoxid, Fluoriden, Vitaminen, Cetylpyridiniumchlorid und Emulgatoren, wie z.B. Natriumlaurylsulfat, Natriumlaurylsarkosinat und Cocamidopropylbetain sowie Süßstoffen wie z.B. Aspartam, Saccharin, Acesulfam-K, Sorbit, Xylit, Cyclamaten (z. B. Natriumcyclamat), Sucralose, Alitam, Neotam, Thaumatin, von Neohesperidin Dihydrochalcon, Maltit, Lactit oder Kaugummi-Massen (GumBasen) oder deren Mischungen, wobei die unangenehmen Geschmackseindrücke bittere oder metallische Geschmackseindrücke sind.

Menthancarbonsäure-N-(4-methoxyphenyl)-amid (WS-12) besitzt eine Struktur gemäß der nachfolgenden Formel (la), vorzugsweise der nachfolgenden Formel (lb), d. h. L-Menthancarbonsäure-N-(4-methoxyphenyl)-amid.

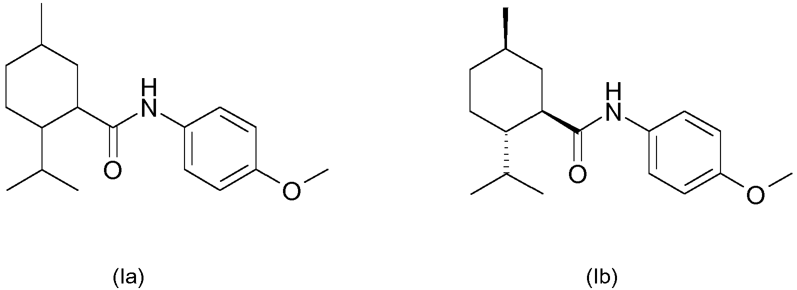

Im Zusammenhang mit der vorliegenden Erfindung ist hierin beschrieben auch eine Zahnputz-Zusammensetzung, vorzugsweise eine Zahnpasta, eine Zahncreme, ein Zahnputzgel oder ein Zahnpulver, zur Anwendung mit einer Zahnbürste, umfassend die folgenden Komponenten:
(i) Putzkörper
(ii) Aromastoff-Komposition umfassend oder bestehend aus:
   a) Menthol,
   b) einer die Bitterkeit des Menthol maskierenden Menge an Menthancarbonsäure-N-(4-methoxyphenyl)-amid (WS-12) sowie
   einem, zwei oder mehr weiteren Aromastoffen, wobei der, mehrere oder sämtliche der weiteren Aromastoffe einen log-K_{ow}-Wert im Bereich von 1,5 bis 5 besitzen.

Überraschenderweise hat sich im Rahmen eigener Untersuchungen herausgestellt, dass Menthancarbonsäure-N-(4-methoxyphenyl)-amid (WS-12), vorzugsweise L-Menthancarbonsäure-N-(4-methoxyphenyl)-amid bei Einsatz in ausreichender Menge in der Lage ist, die Bitterkeit von Menthol in einer Aromastoff-Komposition einer hierin beschriebenen Zahnputz-Zusammensetzung zu maskieren. In den besagten Untersuchungen hat sich gezeigt, dass die zum Maskieren erforderliche Menge an WS-12 nicht ohne weiteres in eine Zahnputz-Zusammensetzung einzuarbeiten ist. Menthan-3-carbonsäure-N-(4-methoxyphenyl)-amid ist eine kristalline Substanz mit einem Schmelzpunk von 177°C, die relativ schwerlöslich ist. Um die Maskierung des Menthols in ausreichendem Maße zu bewirken, muss WS-12 in der Aromastoff-Komposition der Zahnputz-Zusammensetzung in ausreichender Menge gelöst sein. In eigenen Voruntersuchungen hatte sich gezeigt, dass in bestimmten üblichen Aromastoff-Kompositionen die Löslichkeit des WS-12 so niedrig ist, dass nur noch eine nicht ausreichende Maskierung (Reduktion bzw. Verminderung) der negativen Geschmackseindrücke des Menthols erreicht werden kann. Überraschenderweise hat sich jedoch gezeigt, dass bei Anwesenheit der hierin beschriebenen einzusetzenden Komponente (ii) c) d. h. einem, zwei oder mehr weiteren Aromastoffen, wobei der, mehrere oder sämtliche der weiteren Aromastoffe einen log-K_{ow}-Wert von 1,5 bis 5 besitzen, die Löslichkeit von WS-12 insoweit erhöht wird, dass eine die Bitterkeit des Menthols maskierende Menge an WS-12 gut und dauerhaft in die Zahnputz-Zusammensetzung eingearbeitet werden kann.

Das Ergebnis ist eine Zahnputz-Zusammensetzung mit einer Reihe von Vorteilen, die zum Teil weiter unten eingehend erläutert werden. Insbesondere aber handelt es sich um eine Zahnputz-Zusammensetzung, in der trotz der Anwesenheit (beträchtlicher Mengen) von Menthol die Bitterkeit des Menthols nicht mehr störend wirkt, weil sie durch die gleichzeitige Anwesenheit von WS-12 maskiert ist. Das Menthol kann somit beispielsweise seiner ursprünglichen Bedeutung gemäß die negativen Geschmackseindrücke der üblichen in Zahnputz-Zusammensetzungen eingesetzten Wirkstoffe maskieren, ohne dass dabei die negativen geschmacklichen Aspekte des Menthols als nachteilig verbleiben.

Erstmals wurde Menthancarbonsäure-N-(4-methoxyphenyl)-amid (WS-12) als möglicher Kühlwirkstoff beschrieben in US 4,150,052.

In J. Soc. Cosmet. Chem. 1978, 29, 185-200 wurden die Ergebnisse einer Studie an ca. 1200 Verbindungen präsentiert, bei der die Verbindungen L-Menthancarbonsäure-N-ethylamid (WS-3) und auch L-Menthancarbonsäure-N-(4-methoxyphenyl)-amid (WS-12) als Kühlwirkstoffe identifiziert wurden.

Menthancarbonsäure-N-(4-methoxyphenyl)-amid (WS-12) ist somit schon seit längerem als möglicher Kühlwirkstoff bekannt, wurde bisher jedoch in der Praxis nicht in relevantem Umfang als Kühlwirkstoff eingesetzt.

In DE 2503555 wird eine geschmacksverstärkende Wirkung von Menthancarbonsäure-N-(4-methoxyphenyl)-amid in Kaffeezubereitungen, Pulvertee und Orangensaft beschrieben und GB 1351762 behandelt den Einsatz von u.a. Menthancarbonsäure-N-(4-methoxyphenyl)-amid in Tabakwaren.

Die Synthese von WS-12 ist in US 4,178,459, US 4,150,052 sowie GB 1351761 beschrieben.

Die Kühlwirkung von Menthancarbonsäure-N-(4-methoxyphenyl)-amid und in diesem Zusammenhang seine den physiologischen Kühlrezeptor TRPM8 aktivierende Funktion werden in WO 2005/020897 A2 (Dendreon Corporation) und WO 2005/002582 A2 (Genentech Inc.) beschrieben.

In EP 2 068 150 wurden verschiedene Kühlwirkstoffe auf ihre Kühlwirkung hin untersucht. Dabei wurden die relativen Kühlstärken im Vergleich zu L-Menthol bestimmt, wobei L-Menthol eine Stärke von 100 zugeordnet wurde. Gemäß EP 2 068 150 liegt die Kühlstärke von L-Menthancarbonsäure-N-ethylamid (WS-3) und von L-Methancarbonsäure-N-(4-methoxyphenyl)-amid (WS-12) jeweils bei 150. Jedoch wurde N^{α}-(L-Menthancarbonyl)-glycinethylester (WS-5) mit 400 als deutlich stärker kühlend bewertet.

In WO 2006/103401 A2 wird die Kühlwirkung von Menthancarbonsäure-N-(4-methoxyphenyl)-amid an der Oberlippe mit bis zu einer halben Stunde andauernd beschrieben. Die Kühlwirkung wird als geringer im Vergleich zu WS-5 beschrieben. Es wird bemerkt, dass dieser Sachverhalt im Gegensatz zu den Versuchen am TRPM8-Kälterezeptor steht, an dem WS-12 eine stärkere Aktivität zeigt als WS-5.

Die Tatsache, dass Menthancarbonsäure-N-(4-methoxyphenyl)-amid am Kühlrezeptor TRPM8 eine der stärksten Kühlsubstanzen ist, wird ebenfalls in Cell Calcium (42) 2007, 618 und in Cell Calcium (41), 2007, 285 sowie in Pak. J. Pharm. Sci. Vol. 21, 4, 2008, 370 beschrieben.

In US 2005/0187211 A1 wird unter dem Namen 'CPS-112' die Kühldauer von Menthancarbonsäure-N-(4-methoxyphenyl)-amid mit unter einer Stunde auf der Philtrumsfalte (Oberlippe) angegeben. In US 2007/0053834A1 wird der Schwellenwert von WS-12 für ein Kältegefühl auf der Zunge beschrieben.

Die Wasserlöslichkeit von Menthancarbonsäure-N-(4-methoxyphenyl)-amid ist in US 2007/0155755 A1 beschrieben.

Die Verwendung von Menthancarbonsäure-N-(4-methoxyphenyl)-amid als Kühlsubstanz auf der Haut (sinnlich wahrnehmbares Signal) wird beschrieben in US 2007/0233026 A1.

WO 2008/015403 A1 offenbart die Verwendung von Menthancarbonsäure-N-(4-methoxyphenyl)-amid in einem Medikament gegen chronische Schmerzen.

Auch in WO 2008/138162 und in EP 2 033 688 wird jeweils im Rahmen der dort beschriebenen Erfindung der Einsatz von Menthancarbonsäure-N-(4-methoxyphenyl)-amid als möglicher Kühlwirkstoff beschrieben.

Zu verweisen ist im Zusammenhang mit der vorliegenden Erfindung auch auf US-A-5,695,746, WO-A-96/17524, US-A-2009/0148938 und GB-A-1 457 671, die sich mit der Mundpflege dienenden Zusammensetzungen und/oder der Substanz WS-12 beschäftigen.

In US 5,009,893, US 5,372,824 und WO 96/17524 werden bestimmte essbare Zusammensetzungen (insbesondere Kaugummi-Zusammensetzungen) offenbart, in denen Menthol sowie bestimmte N-substituierte Amide der Menthan-3-carbonsäure vorliegen. Diese Zusammensetzungen sollen eine langanhaltende den Atem erfrischende Wahrnehmung ohne Bitterkeit zeigen. Als besonders geeignetes Amid wird dabei das Menthan-3-carbonsäure-N-ethylamid genannt.

In eigenen Untersuchungen erwies sich der Einsatz von Menthan-3-carbonsäure-N-ethylamid, welches im Zentrum der Überlegungen gemäß den drei soeben diskutierten Patentschriften stand, in Zahnputz-Zusammensetzungen als weitgehend wirkungslos. Insbesondere führte in eigenen Untersuchungen der Zusatz von Menthan-3-carbonsäure-N-ethylamid zu einer üblichen Menthol enthaltenden Zahnputz-Zusammensetzung, nicht zu einer signifikanten Reduktion oder Maskierung der von Menthol verursachten Bitterkeit bzw. Schärfe.

Möglicherweise ist die Wirkungslosigkeit von Menthan-3-carbonsäure-N-ethylamid in Zahnputz-Zusammensetzungen (und insbesondere in Zahnpasta) in dem unterschiedlichen Aufbau der Zahnputz-Zusammensetzung im Vergleich mit einem Kaugummi begründet. Zahnpasten sind viskose Suspensionen, die beim Zähneputzen durch die Verdünnung mit Speichel schnell dünnflüssig werden. Übliche Viskositätswerte für Zahnpasten liegen im Bereich zwischen 50 und 400 Pa s bei 25°C.

Hierin beschriebene Zahnpasten (als Beispiel einer besonders bevorzugten Zahnputz-Zusammensetzung) weisen vorzugsweise Viskositätswerte im Bereich von 10 bis 500 Pa s, bevorzugt im Bereich von 50 bis 450 Pa s auf, jeweils gemessen bei 25°C und bei einer Scherrate D=10 s⁻¹, beispielsweise gemessen mit einem Brookfield® Viskosimeter gemäß DIN 53018.

Hierin beschriebene Zahnpasten mit Viskositätswerten im Bereich von etwa 10 bis 100 Pa s werden als flüssige Zahnpasten bezeichnet, hierin beschriebene viskosere Zahnpasten mit einer marktüblichen pastösen Konsistenz besitzen hingegen Viskositätswerte im Bereich von 150 bis etwa 380 Pa s, bevorzugt im Bereich von 180 bis 350 Pa s.

Kaugummi hingegen besteht überwiegend aus einer hochviskosen Masse, deren Zähigkeit auch bei länger andauerndem Kauen kaum geringer wird und die sich auch nicht während des Kauvorganges auflöst.

Dementsprechend sind die Viskositäten von üblicherweise eingesetzten Gum-Basen (die die Grundlage von Kaugummi darstellen) höher als die Viskositäten von Zahnpasten. Die Messung der Viskosität von Gum-Basen ist nur bei erhöhter Temperatur möglich; üblicherweise werden für solche Gum-Basen Viskositätswerte von ca. 200 Pa s bei 90°C angegeben. Aufgrund des unterschiedlichen Aufbaus und der unterschiedlichen weiteren Eigenschaften von Kaugummis und Zahnpasten werden vermutlich Aromastoffe aus der Zahnpasta und dem Kaugummi in entscheidend unterschiedlicher Weise freigesetzt. Im Kaugummi werden die Aromastoffe von der zähen Kaugummi-Masse viel stärker zurückgehalten und erst langsam freigesetzt. Im Gegensatz dazu werden Aromastoffe in einer Zahnpasta eher sehr früh, das heißt unmittelbar in ihrer ganzen Stärke, beim Zähneputzen freigesetzt und daher im Vergleich als sehr viel stärker empfunden. Es erscheint daher aus heutiger Perspektive nachvollziehbar, dass in Kaugummi durch den Zusatz kleinerer Mengen an Modifikatoren bereits Effekte erzielt werden können, während dies in Zahnpasta und anderen Zahnputz-Zusammensetzungen nicht der Fall ist.

Überdies muss auch in Betracht gezogen werden, dass Kaugummi üblicherweise sehr viel größere Mengen an süß schmeckenden Stoffen enthält als Zahnputz-Zusammensetzungen. Diese süß schmeckenden Stoffe sind bereits für sich in der Lage, den scharf-bitteren Geschmack von Menthol abzumildern.

Hierin beschriebene Zahnputz-Zusammensetzungen besitzen eine Reihe von Vorteilen aufgrund der Anwesenheit von WS-12 als Maskierungsmittel für Menthol:
In einer hierin beschriebenen Zahnputz-Zusammensetzung kann der Anteil an Menthol (als Komponente a) einer hierin beschriebenen Aromastoff-Komposition (ii)) vergleichsweise hoch sein, ohne dass die Eigenbitterkeit des Menthols wahr genommen wird oder als störend empfunden wird. Bei Einsatz einer hohen Menge an Menthol wird das Frische- und Kälteempfinden bei der Verwendung der hierin beschriebenen Zahnputz-Zusammensetzung (vorzugsweise Zahnpasta) besonders ausgeprägt und dadurch auch die Wahrnehmung derjenigen unangenehmen Geschmacksempfindungen herabgesetzt, welche durch die üblicherweise vorhandenen Zahnputz-Wirkstoffe verursacht werden (siehe dazu die obigen allgemeinen Anmerkungen).

Besonders relevante hierin beschriebene Zahnputz-Zusammensetzungen umfassen deshalb Wirkstoffe, welche selbst unangenehme Geschmacksempfindungen verursachen, also insbesondere Triclosan, Kalium- oder Zinkcitrat, Zinnchlorid, Zinndichlorid, Zinnfluorid, Aminfluorid oder Cetylpyridiniumchlorid. Hierin beschriebene Zahnputz-Zusammensetzungen umfassen zudem vorzugsweise einen beträchtlichen Anteil von Menthol, der vorzugsweise ausreicht, um die als unangenehm empfundenen Geschmacksnoten der in den Zahnputz-Zusammensetzungen enthaltenen Wirkstoffe vollständig oder teilweise zu maskieren. Bevorzugte hierin beschriebene Zahnputz-Zusammensetzungen enthalten Menthol in einem Bereich von 0,15 bis 1,3 Gew.-% bezogen auf das Gesamtgewicht der bevorzugten Zahnputz-Zusammensetzung. Besonders bevorzugte hierin beschriebene Zahnputz-Zusammensetzungen enthalten 0,4 bis 0,9 Gew.-% Menthol bezogen auf das Gesamtgewicht der besonders bevorzugten Zahnputz-Zusammensetzung.

Das eingesetzte Menthol kann dabei als Bestandteil einer Aromastoff-Komposition natürlichen Ursprungs eingesetzt sein, insbesondere kann es sich um eine PfefferminzölKomposition mit einem Anteil an Menthol handeln. Anstelle von oder zusätzlich zu einer solchen Aromastoff-Komposition natürlichen Ursprungs kann aber auch reines (beispielsweise synthetisches) Menthol zugesetzt werden. Der Fachmann spricht insoweit von linearen Zusätzen von Menthol zu einer Basismischung. Es versteht sich, dass eine hierin beschriebene Zahnputz-Zusammensetzung einen vergleichsweise hohen Anteil an einer natürlichen Aromastoff-Komposition umfassend Menthol und/oder an (linear zugesetztem) Menthol enthalten kann, weil die Bitterkeit des Menthols durch die eingesetzte Menge an WS-12 maskiert wird.

In einer hierin beschriebenen Zahnputz-Zusammensetzung müssen keine weiteren ggf. streng schmeckenden Aromastoffe vorhanden sein, welche in üblichen Zahnputz-Zusammensetzungen dazu dienen, die Bitterkeit von Menthol zu maskieren. Ihre Anwesenheit in hierin beschriebenen Zahnputz-Zusammensetzungen ist somit zwar nicht ausgeschlossen, jedoch auch nicht bevorzugt. Insbesondere kann eine hierin beschriebene Zahnputz-Zusammensetzung zwar Anethol umfassen (siehe hierzu die obige Diskussion), die (Haupt-) Bedeutung des Anethols ist dann aber vorzugsweise nicht das Maskieren des bitteren Geschmacks des Menthols.

In den hierin beschriebenen Zahnputz-Zusammensetzungen kann insbesondere bei Abwesenheit von strengschmeckenden Aromastoffen die Anwesenheit anderer Aromen mit insbesondere wohlschmeckenden Geschmacksnoten vorgesehen sein. Insbesondere sind hierin beschriebene Zahnputz-Zusammensetzungen vorteilhaft, in denen Geschmacksnoten der Richtungen Frucht, Zimt, Wintergrün, Eukalyptus, Krauseminz (Spearmint) und Pfefferminz vorliegen. Die jeweils eingesetzte Menge an Geschmacksstoffen, welche für die genannten Geschmacksnoten verantwortlich sind, kann dabei gering sein, denn aufgrund der Anwesenheit von WS-12 als Bittermaskierer für Menthol kann auf die Anwesenheit von z. B. Anethol (als Bittermaskierer mit eigenen unerwünschten Eigenschaften) verzichtet werden. Im Einzelfall kann aufgrund der Anwesenheit der vorstehend genannten oder anderer wohlschmeckender Geschmacksnoten, die vom Verbraucher überwiegend als sehr angenehm geschätzt werden, eine unangenehme Geschmacksempfindung, die von eingesetzten primären Wirkstoffen stammt, weiter reduziert werden.

Neben den vorstehend diskutierten Vorteilen einer Zahnputz-Zusammensetzung ist ein weiterer Vorteil von besonderer wirtschaftlicher Relevanz. Hierin beschriebene Zahnputz-Zusammensetzungen vermitteln nämlich ein besonders langes, gegenüber Zahnputz-Zusammensetzungen aus dem Stand der Technik deutlich verlängertes kühlendes Frischeempfinden, das sich beispielsweise nach dem Zähneputzen mit einer hierin beschriebenen Zahnpasta einstellt. Dieses langanhaltende, kühlende Frischeempfinden hängt von der Anwesenheit von WS-12 in der hierin beschriebenen Zahnputz-Zusammensetzung ab.

Im Gegensatz zu hierin beschriebenen Zahnputz-Zubereitungen umfassend WS-12 führen Zahnputz-Zusammensetzungen mit konventionellen Kühlwirkstoffen, wie sie z.B. in in M. Erman, Perfumer & Flavorist 32(10), 20-35 (2007) oder M. L. Dewis in D. J. Rowe, Chemistry and Technology of Flavors and Fragrances, Blackwell Publishing Ltd, Oxford 2005, p. 212 - 222 beschrieben werden, nur zu einem vergleichsweise viel kürzer empfundenen Frischeempfinden. Vergleichs-Zahnputz-Zusammensetzungen, welche die besagten konventionellen Kühlwirkstoffe enthalten, vermitteln zwar ebenfalls eine Kühlwirkung, die sogar nach etwa 0,5 Minuten relativ schnell einsetzt, die dann aber nach einem Höhepunkt bei drei bis fünf Minuten relativ schnell wieder abflacht, wobei die Kühlung insgesamt für höchstens 30 Minuten deutlich wahrnehmbar ist. In eigenen Untersuchungen hat sich insoweit insbesondere das oben erwähnte Menthyl-3-carbonsäure-N-ethylamid als Kühlwirkstoff erwiesen, der nur für einen relativ kurzen Zeitraum eine Kühlwirkung vermittelt. In eigenen Vergleichsuntersuchungen hat sich überdies gezeigt, dass sich durch eine Erhöhung der eingesetzten Menge an konventionellen Kühlwirkstoffen die Dauer der durch das Kältegefühl erzeugten Frischeempfindung nicht verlängern lässt.

Gemäß eigener Recherchen ist bislang nur eine einzige Verbindung (2-Isopropyl-5-methyl-cyclohexanecarbonsäure-(4-cyanomethyl-phenyl)-amid (CAS 852379-28-3)) bekannt, die sich aufgrund ihrer länger anhaltenden Kühlwirkung in relevanter Weise von herkömmlichen Kühlwirkstoffen unterscheidet (vgl. hierzu S. M. Furrer et al, Chem. Percept 1 (2008), 119 - 126). Es ist für den Fachmann schwierig, Substanzen mit außergewöhnlich langanhaltender Kühlwirkung aufzufinden, da sich die in-vitro durch Rezeptorbindung ermittelte Aktivität mit der Intensität, aber nicht mit der Dauer des Kühlempfindens korrelieren lässt (loc. cit, Seite 120).

Es ist besonders überraschend, dass eine hierin beschriebene Zahnputz-Zusammensetzung regelmäßig eine deutlich verlängerte Frischeempfindung hervorruft, die in den meisten Fällen zumindest doppelt so lange anhält, wie eine Frischeempfindung, welche durch Vergleichs-Zahnputz-Zusammensetzungen hervorgerufen wird, die eine der oben genannten konventionellen Kühlsubstanzen enthält.

Besonders überraschend ist, dass sich mit WS-12 (Menthancarbonsäure-N-(4-methoxyphenyl)-amid) eine befriedigende Kühlwirkung verknüpft mit einem besonders langanhaltendem Frischeempfinden erzielen lässt. Aufgrund ihres hohen Schmelzpunktes von 177°C war der Verbindung eine ungenügende Löslichkeit in Mundpflegearomen und somit eine nicht ausreichende Bio-verfügbarkeit vorhergesagt worden (loc cit., Seiten 121, 122).

In hierin beschriebenen Zahnputz-Zusammensetzungen ergänzen sich somit zwei für die Bedeutung der vorliegenden Erfindung relevante Aspekte in überzeugender Weise:
Zum einen maskiert die eingesetzte Menge an WS-12 die bitteren Geschmackseindrücke des Menthols. Zum anderen vermittelt die Zahnputz-Zusammensetzung aufgrund der Anwesenheit von WS-12 neben Menthol (und neben der weiteren Komponente c)) eine besonders lang anhaltende Frischeempfindung.

In einer hierin beschriebenen Zahnputz-Zusammensetzung kommt der Komponente c) der Aromastoff-Komposition die Aufgabe zu, die zum Zwecke der Bittermaskierung erforderliche Menge WS-12 in der Zahnputz-Zusammensetzung löslich zu machen und damit für die Zwecke der Bittermaskierung zur Verfügung zu stellen. Es hat sich gezeigt, dass in Vergleichs-Zahnputz-Zusammensetzungen, die keine Komponente c) enthalten, die gelöste Menge an WS-12 regelmäßig zu gering ist, als dass sie eine wirksame Maskierung des eingesetzten Menthols bewirken könnte. Erfindungsgemäß wird nicht bzw. nicht ausschließlich ein beliebiger Stoff mit einem log-K_{ow}-Wert im Bereich von 1,5 bis 5 eingesetzt, um die erforderliche Löslichkeit von WS-12 in der Gesamt-Zusammensetzung zu erreichen, sondern es werden zumindest einer, zwei oder mehr Aromastoffe eingesetzt, die einen entsprechenden log-K_{ow}-Wert besitzen. Vorzugsweise wird in einer hierin beschriebenen Zahnputz-Zusammensetzung vollständig auf die Anwesenheit von Substanzen verzichtet, die einen log-K_{ow}-Wert im Bereich von 1,5 bis 5 besitzen und nicht gleichzeitig ein Aromastoff sind. Statt solcher Nicht-Aromastoff-Substanzen ist nämlich der Einsatz von entsprechenden Aromastoffen bevorzugt.

Der log-K_{ow}-Wert ist ein Maß für die Polarität einer Substanz. Mit log-K_{ow} wird der dekadische Logarithmus des Verteilungskoeffizienten einer Substanz zwischen 1-Oktanol (unpolar) und Wasser (polar) bezeichnet.

Der log-K_{ow}-Wert wird dabei in der Praxis rechnerisch ermittelt, wobei die jeweilige molekulare Struktur der Substanz, deren log-K_{ow}-Wert bestimmt werden soll, zugrunde gelegt wird. Im Rahmen des vorliegenden Textes bezeichnet der Begriff "log-K_{ow}-Wert" den anhand der jeweiligen molekularen Struktur berechneten Wert bei Verwendung des Software-Programms "EPIWIN" von P. Howard und W. Meylan [Version 2.2]. Dieses Software-Programm ist erhältlich bei Syracuse Research Corporation, Merrill Lane, Syracuse, NY 13210, USA.

Es ist eine hierin beschriebene Zahnputz-Zusammensetzung bevorzugt, wobei der, mehrere oder sämtliche weiteren Aromastoffe mit einem log-K_{ow}-Wert im Bereich von 1,5 bis 5 ausgewählt sind aus der Gruppe bestehend aus Menthon, Isomenthon, Carvon, 1,2-Dihydrocarvon, Anethol, Piperiton, Menthylacetat, Menthylmethylether, 1,8-Cineol, Zimtaldehyd und Methylsalicylat, vorzugsweise aus der Gruppe bestehend aus Menthon, Isomenthon, Carvon und Piperiton.

Als Komponente c) einer Aromastoff-Komposition (ii) einer hierin beschriebenen Zahnputz-Zusammensetzung kann also ein einziger oder können zwei oder mehr Aromastoffe der genannten Gruppe in Kombination miteinander eingesetzt werden.

Für bevorzugte Aromastoffe, die als Komponente oder in Komponente c) der hierin beschriebenen Zahnputz-Zusammensetzung eingesetzt werden können, sind nachfolgend die mit dem Programm EPIWIN ermittelten log-K_{ow}-Werte in Klammern angegeben:
Menthon (2,87), Isomenthon (2,87), Carvon (3,07), 1,2-Dihydrocarvon (2,86), Anethol (3,39), Piperiton (3,07), Menthylacetat (4,39), 1,8-Cineol (3,13), Zimtaldehyd (1,82) und Methylsalicylat (2,60).

Bevorzugte Enantiomere der besonders bevorzugten Aromastoffe des Bestandteils (c) sind (-)-Menthon (2,87), (+)-Isomenthon (2,87), (-)-Carvon (3,07) und (-) Piperiton (3,07).

Bevorzugt ist die Verwendung von Aromastoffen der vorstehend angegebenen Gruppe in bzw. als Komponente c) einer hierin beschriebenen Zahnputz-Zusammensetzung, wobei der log-K_{ow}-Wert des jeweiligen Aromastoffes im Bereich von 1,8 bis 4,5 liegt.

Ganz besonders bevorzugt ist eine hierin beschriebene Zahnputz-Zusammensetzung wie vorstehend definiert, wobei der oder mindestens einer der weiteren Aromastoffe Menthon ist.

Als überraschend nicht nachteilig erwiesen hat es sich dabei, dass die genannten Aromastoffe der Komponente c) in der hierin beschriebenen Zahnputz-Zusammensetzung ihrerseits einen Bittergeschmack besitzen können. So besitzen beispielsweise die besonders bevorzugten Aromastoffe Menthon und Isomenthon, die in "Perfume and Flavor Chemicals" von Steffen Arctander (veröffentlicht 1969) unter den Substanznummern 1843 (Menthon) bzw. 1844 (Isomenthon) beschrieben werden, einen bitteren Geschmack.

In derartigen Zahnputz-Zusammensetzungen, die neben dem bitterschmeckenden Stoff Menthol als Komponente c)) einen, zwei oder weitere bitterschmeckende Aromastoffe umfassen, maskiert die eingesetzte Menge an WS-12 nicht nur die Bitterkeit des Menthols, sondern auch (zumindest teilweise) die Bitterkeit des oder der besagten weiteren Aromastoffe. Der Fachmann wird anhand weniger Vorversuche ermitteln, welche Mengen an WS-12 (als Komponente b)) und an dem einen, den zwei oder weiteren Aromastoffen er einsetzen sollte, damit ein bestimmtes Höchstmaß an Bitterkeit nicht überschritten wird. Er wird dabei insbesondere berücksichtigen, ob ein einzusetzender weiterer Aromastoff selbst einen bitteren Geschmackseindruck vermittelt oder lediglich eingesetzt wird, um eine ausreichende Löslichkeit von WS-12 in der hierin beschriebenen Zahnputz-Zusammensetzung zu vermitteln.

Sind in einer hierin beschriebenen Zahnputz-Zusammensetzung neben Menthol noch eine oder weitere bitterschmeckende Substanzen enthalten, deren die Bitterkeit betreffende Effekte nicht unterscheidbar sind, so wird die eingesetzte Menge an WS-12 als "die Bitterkeit des Menthols maskierende Menge" bezeichnet, wenn die Gesamtbitterkeit der Zahnputz-Zusammensetzung aufgrund der Anwesenheit von WS-12 reduziert wird.

Vorzugsweise handelt es sich bei dem einen, den zwei oder weiteren Aromastoffen mit einem log-K_{ow}-Wert von 1,5 bis 5 um aprotische Aromastoffe.

Die Aromastoffe der Komponente c) einer hierin beschriebenen Zahnputz-Zusammensetzung können in Form eines etherischen Öles eingesetzt werden, welches diese Stoffe enthält. Besonders bevorzugt ist der kombinierte Einsatz von reinen Aromastoffen gemeinsam mit Aromastoffen, die in einem oder mehreren etherischen Ölen vorliegen. Die so kombinierten Aromastoffe bilden dann gemeinsam die Komponente c) einer hierin beschriebenen Zahnputz-Zusammensetzung.

Eine hierin beschriebene Zahnputz-Zusammensetzung ist kein Kaugummi; sie enthält deshalb allenfalls 0 bis 10 Gew.-% einer Kaugummi-Base bezogen auf die Gesamtmasse der Zahnputz-Zusammensetzung, vorzugsweise 0 bis 3 Gew.-% einer Kaugummi-Base, besonders bevorzugt keine Kaugummi-Base (0 Gew.-%). Gleichzeitig oder alternativ umfasst eine bevorzugte hierin beschriebene Zahnputz-Zusammensetzung allenfalls eine oder mehrere Elastomere in einer Gesamtmenge von 0 bis 20 Gew.-%, bezogen auf die Gesamtmasse der Zahnputz-Zusammensetzung (vgl. hierzu Beispiel 11 unten, worin Poloxamer 407 und Polyethylenglycol als Elastomere aufzufassen sind).

In eigenen Untersuchungen hat sich gezeigt, dass insbesondere bei Anwesenheit von WS-12 in Kombination mit weiteren Aromastoffen, wie sie zur Aromatisierung in klassischen Mundhygieneapplikationen verwendet werden, ein fundamental von den bereits bekannten Kühlsubstanzen verschiedenes Kälteprofil vermittelt wird. Abhängig von der genauen Zusammensetzung ist beispielsweise beim Putzen der Zähne mit einer hierin beschriebenen Zahnpasta, die ein Aroma mit WS-12 enthält, eine Kälteempfindung von bis zu 2 Stunden wahrnehmbar.

Die besonders langanhaltende Kälteempfindung wird insbesondere durch hierin beschriebene Zahnputz-Zusammensetzungen erreicht, in denen WS-12 mit 1,8-Cineol (Eucalyptol), Menthon, Carvon, Zimtaldehyd und/oder Methylsalicylat kombiniert wird. Es handelt sich hierbei um Aromastoffe, wie sie als Bestandteil der Komponente c) bereits weiter oben diskutiert wurden. Die besagten Aromastoffe erfüllen somit in einer bevorzugten hierin beschriebenen Zahnputz-Zusammensetzung zwei Aufgaben: zum einen erhöhen sie die Löslichkeit von WS-12 soweit, dass eine Menge von WS-12 eingesetzt werden kann, die ausreicht, die Bitterkeit des Menthols zu maskieren. Zum anderen verlängern sie die Kühlwirkung der hierin beschriebenen Zahnputz-Zusammensetzung noch weiter.

Es versteht sich, dass die genannten Aromastoffe in Form von Aromenmischungen eingesetzt werden können. Bevorzugt ist daher eine hierin beschriebene Zahnputz-Zusammensetzung umfassend
- Eucalyptusaromen enthaltend Eucalyptol;
- Pfefferminzaromen enthaltend Menthon;
- Spearmintaromen enthaltend Carvon;
- Zimtaromen, enthaltend Zimtaldehyd;
- Wintergrünaromen enthaltend Methylsalicylat.

Eine hierin beschriebene Zahnputz-Zusammensetzung wie vorstehend definiert (insbesondere wie vorstehend als bevorzugt bezeichnet) enthält vorzugsweise zusätzlich weitere Bestandteile bzw. Komponenten. Bevorzugt ist es, wenn eine solche Zahnputz-Zusammensetzung
- zusätzlich Zuckerersatzstoffe enthält, vorzugsweise nicht-kariogene Zuckerersatzstoffe,
   und/oder
- frei ist von Saccharose, Glucose und Fructose, vorzugsweise ganz frei von kariogenen Zuckern,
   und/oder
- zusätzlich Feuchthaltemittel enthält, vorzugsweise einen feuchthaltenden Zuckereralkohol, vorzugsweise Sorbit oder Xylitol,
   und/oder
- zusätzlich Verdickungsmittel enthält,
   und/oder
- zusätzlich ein oder mehrere oberflächenaktive Substanzen enthält, vorzugsweise Natriumlaurylsulfat,
   und/oder
- zusätzlich ein oder mehrere antimikrobielle Wirkstoffe enthält, vorzugsweise ausgewählt aus der Gruppe bestehend aus Hydroxybenzoesäureester, Parabene, Triclosan und/oder
- zusätzlich ein oder mehrere Farbstoffe enthält
   und/oder
- zusätzlich einen oder mehrere Antikaries-Wirkstoffe enthält, vorzugsweise ein oder mehrere Fluoride enthält
   und/oder
- zusätzlich einen oder mehrere weitere physiologische Kühlwirkstoffe enthält.

Zum Einsatz in hierin beschriebenen Zahnputz-Zusammensetzungen besonders bevorzugt sind Zuckerersatzstoffe, bei denen es sich um Süssungsmittel wie Süßstoffe und Zuckeraustauschstoffe oder Mischungen davon handelt, die ausgewählt sind aus der Gruppe bestehend aus:
- Süßstoffen wie insbesondere Acesulfam-K, Aspartam, Cyclamat (sowie dessen Na- und Ca-Salze), Neohesperidindihydrochalcon, Sucralose und Saccharin (sowie dessen Na-, K- und Ca-Salze). Pflanzliche Süßstoffe können ebenfalls verwendet werden, wie beispielsweise Glycyrrhicin und Thaumatin. Besonders bevorzugt sind Acesulfam-K, Aspartam, Cyclamat, Na-Cyclamat, Saccharin, Na-Saccharin und Sucralose;
   und
- Zuckeraustauschstoffen, vorzugsweise Zuckeralkoholen, insbesondere Isomaltitol (E 953), Lactitol (E 966), Maltitol, Mannitol (E 421), Sorbitol (E 420), Xylitol (E 967) sowie Gemischen daraus.

Bei Kombinationen von WS-12 (als Komponente b) einer hierin beschriebenen Zahnputz-Zusammensetzung) mit Diolen (als zusätzlichem Zuckerersatzstoff in einer hierin beschriebenen Zahnputz-Zusammensetzung), insbesondere in Kombination mit 1,2-(Alkan-)Diolen, dabei vorzugsweise 1,2-Propylenglycol, 1,2-Pentandiol, 1,2-Octandiol), Triolen, dabei insbesondere Glycerin, oder Polyolen, dabei vorzugsweise Zuckeralkoholen, wie Isomaltitol (E 953), Lactitol (E 966), Maltitol, Mannitol (E 421), Sorbitol (E 420), Xylitol (E 967) sowie Mischungen dieser Zuckeralkohole, wurde zudem überraschenderweise gefunden, dass die Kühlwirkung von WS-12 signifikant verlängert ist. Die bevorzugte Gesamtmenge an Diolen, Triolen und Polyolen in einer hierin beschriebenen Zahnputz-Zusammensetzung liegt vorzugsweise im Bereich von 0,5 bis 50 Gew.-%, bevorzugt im Bereich von 2 bis 45 Gew.-%, am meisten bevorzugt im Bereich von 5 bis 40 Gew.-%, jeweils bezogen auf das Gesamtgewicht der hierin beschriebenen Zahnputz-Zusammensetzung.

Eine bevorzugte hierin beschriebene Zahnputz-Zusammensetzung ist frei von Saccarose, Glucose und Fructose und vorzugsweise ganz frei von kariogenen Zuckern. Dies gilt natürlich insbesondere dann, wenn eine bevorzugte hierin beschriebene Zahnputz-Zusammensetzung Zuckerersatzstoffe enthält, vorzugsweise nicht-kariogene Zuckerersatzstoffe wie vorstehend diskutiert. Der Fachmann wird im Einzelfall entscheiden, ob gänzlich auf kariogene Zucker in einer hierin beschriebenen Zahnputz-Zusammensetzung verzichtet werden soll bzw. kann, und er wird entsprechende Mengen von Zuckerersatzstoffen vorsehen, um den Bedürfnissen der Konsumenten zu entsprechen.

Eine bevorzugte hierin beschriebene Zahnputz-Zusammensetzung (insbesondere eine vorstehend bereits als bevorzugt bezeichnete hierin beschriebene Zahnputz-Zusammensetzung) enthält zusätzlich Feuchthaltemittel (z.B. Glycerin). Vorzugsweise handelt es sich bei solchen Feuchthaltemitteln um feuchthaltende Zuckeralkohole wie beispielsweise Sorbit oder Xylitol. Der Einsatz eines feuchthaltenden Zuckeralkohols entspricht somit gleichzeitig dem Einsatz eines Zuckerersatzstoffes und eines Feuchthaltemittels.

Eine bevorzugte hierin beschriebene Zahnputz-Zusammensetzung (insbesondere eine vorstehend bereits als bevorzugt bezeichnete hierin beschriebene Zahnputz-Zusammensetzung) enthält zusätzlich Verdickungsmittel, vorzugsweise ausgewählt aus der Gruppe bestehend aus: Polyethylenglycole, Laponite®, Carboxymethylcellulose, Xanthan, Tragant, Johannisbrotkernmehl, Gellan, Guarkernmehl, Gummi arabicum, Carrageenane, Alginsäure, Alginate, Pektine, pyrogene Kieselsäure, Bentonite, Magnesium-Aluminium-Silicate sowie Mischungen daraus. Als Verdickungsmittel zum Einsatz in einer hierin beschriebenen Zahnputz-Zusammensetzung besonders geeignet sind unter den genannten die Hydrokolloide.

Eine bevorzugte hierin beschriebene Zahnputz-Zusammensetzung (insbesondere eine vorstehend bereits als bevorzugt bezeichnete hierin beschriebene Zahnputz-Zusammensetzung) umfasst zusätzlich ein oder mehrere oberflächenaktive Substanzen, vorzugsweise Natriumlaurylsulfat, Natriumlaurylsarcosinat und/oder Cocamidopropylbetain, besonderes bevorzugt Natriumlaurylsulfat. Allgemein können in einer hierin beschriebenen Zahnputz-Zusammensetzung anionische, zwitterionische, amphotere, nichtionische Tenside oder eine Kombination mehrerer dieser Tenside eingesetzt werden.

Eine hierin beschriebene Zahnputz-Zusammensetzung (insbesondere eine vorstehend bereits als bevorzugt bezeichnete hierin beschriebene Zahnputz-Zusammensetzung) enthält vorzugsweise zusätzlich einen oder mehrere antimikrobielle Wirkstoffe, die vorzugsweise ausgewählt sind aus der Gruppe bestehend aus Hydroxybenzoesäureester, Parabene und Triclosan. Die antimikrobiellen Wirkstoffe können z. B. die Funktion eines Konservierungsmittels oder eines Antiplaque-Wirkstoffes haben. Als Hydroxybenzoesäureester können insbesondere eingesetzt werden p- Hydroxybenzoesäuremethyl-,-ethyl- oder -propylester. Anstelle der vorstehend bereits genannten antimikrobiellen Wirkstoffe oder zusätzlich zu diesen werden in manchen Fällen vorzugsweis Natriumsorbat, Natriumbenzoat, Bromchlorophen, Phenyl-Salicylsäureester, Biguanide(z.B. Chlorhexidin) und/oder Thymol eingesetzt.

Als antimikrobielle Wirkstoffe eingesetzt werden in manchen Fällen auch vorzugsweise bakterizide Substanzen, wie z. B. Phenole, Resorcine, Bisphenole, Salicylanilide und deren halogenierte Derivate, halogenierte Carbanilide und p- Hydroxybenzoesäureester. Das besonders bevorzugt einzusetzende Triclosan ist ein halogenierter Diphenylether, nämlich 2,4,4'-Trichlor-2'-hydroxydiphenylether. Anstelle von oder zusätzlich zu Triclosan können andere halogenierte Diphenylether eingesetzt werden, z. B. 2,4-Dichlor-2'-hydroxydiphenylether, 4,4'-Dichlor-2'-hydroxydiphenlyether oder 2,4,4'-Tribrom-2'-hydroxydiphenylether. Halogenierte Diphenylether wie z. B. Triclosan werden bevorzugt in einer Gesamtmenge von 0,01 bis 1 Gew.-% in einer hierin beschriebenen Zahnputz-Zusammensetzung eingesetzt. Triclosan selbst wird besonders bevorzugt in einer Menge von 0,01 bis 0,3 Gew.-% eingesetzt.

Eine hierin beschriebene Zahnputz-Zusammensetzung (insbesondere eine vorstehend bereits als bevorzugt bezeichnete hierin beschriebene Zahnputz-Zusammensetzung) enthält vorzugsweise zusätzlich ein oder mehrere Farbstoffe. Diese Farbstoffe sind vorzugsweise in einer Menge vorhanden, die ausreicht, um der entsprechenden Zahnputz-Zusammensetzung eine gewünschte Farbe zu vermitteln. Die eingesetzte Menge typischer Farbstoffe liegt deshalb etwa im Bereich von 2 - 5 Gew.-%, bezogen auf das Gesamtgewicht der Zahnputz-Zusammensetzung. Ein Anteil von ungefähr 3 Gew.-% an Farbstoffen ist häufig geeignet. Die eingesetzten Farbstoffe können natürliche Nahrungsmittelfarben sein sowie Farbstoffe, die sich für Nahrungsmittel-, Arzneimittel- und kosmetische Anwendungen eignen. Geeignete Farbstoffe sind als FD & C Farbstoffe und Färbemittel bekannt. Die einzusetzenden Farbstoffe sind vorzugweise wasserlöslich. Beispiele geeigneter Farbstoffe sind der Indigofarbstoff, welcher als FD & C Blau No.1 und FD & C Gelb No.10 bekannt ist. Eine vollständige Auflistung von FD & C-Farbstoffen und ihren entsprechenden Strukturen findet sich in der Kirk-Othmer, Encyclopedia of Chemical Technology, 3rd Edition, Volume 5, Seiten 857 bis 884.

Einzusetzende Farbstoffe können auch die Funktion eines Trübungsmittels erfüllen. So kann z. B. Titandioxid in Mengen von bis zu vorzugsweise 3 Gew.-%, bevorzugt 1 Gew.-%, ganz besonders bevorzugt 0,5 Gew.-% in eine hierin beschriebene Zahnputz-Zusammensetzung eingearbeitet sein, bezogen auf das Gesamtgewicht der Zahnputz-Zusammensetzung.

Eine hierin beschriebene Zahnputz-Zusammensetzung (insbesondere eine vorstehend bereits als bevorzugt bezeichnete hierin beschriebene Zahnputz-Zusammensetzung) enthält vorzugsweise zusätzlich einen oder mehrere Antikaries-Wirkstoffe. Bei dem oder diesen Antikaries-Wirkstoffen handelt es sich üblicherweise um einen oder mehrere Fluoride, wobei organische oder anorganische Fluoride eingesetzt werden können. Bevorzugte Fluoride zum Einsetzen in einer hierin beschriebenen Zahnputz-Zusammensetzung sind beispielsweise Natriumfluorid, Kaliumfluorid, Natriummonofluorphosphat, quartäre Ammoniumfluoride und Natriumfluorosilikat. Auch Zinkfluorid und Zinn-(II)-fluorid werden in Einzelfällen bevorzugt eingesetzt. Bevorzugt ist der Einsatz einer Gesamtmenge von 0,01 bis 0,2 Gew.-% Fluor in Form einer der genannten oder in Form einer anderen Fluorid in geeigneter Weise zur Verfügung stellenden Verbindung.

Eine hierin beschriebene Zahnputz-Zusammensetzung (insbesondere eine vorstehend bereits als bevorzugt bezeichnete hierin beschriebene Zahnputz-Zusammensetzung) enthält vorzugsweise zusätzlich einen oder mehrere weitere physiologische Kühlwirkstoffe. Der bzw. diese weiteren physiologischen Kühlwirkstoffe werden dabei vorzugsweise ausgewählt aus der Gruppe bestehend aus: Menthylether (z.B. (I-Menthoxy)-1,2-propandiol, (I-Menthoxy)-2-methyl-1,2-propandiol, I-Menthyl-methylether), Menthylester (z.B. Menthylformiat, Menthylacetat, Menthylisobutyrat, Menthyllactate, vorzugsweise L-Menthyllactat, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylalkylcarbonate, Menthylpropylenglycolcarbonat, Menthylethylenglycolcarbonat, Menthylglycerincarbonat oder deren Mischungen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (z.B. Menthancarbonsäure-N-ethylamid [WS-3], N^{α}-(Menthancarbonyl)glycinethylester [WS-5], Menthancarbonsäure-N-(4-cyanophenyl)amid, Menthancarbonsäure-N-(alkoxyalkyl)amide), Menthon und Menthonderivate (z.B. Menthonketale wie L-Menthonglycerinketal), 2,3-Dimethyl-2-(2-propyl)-butansäurederivate (z.B. 2,3-Dimethyl-2-(2-propyl)-butansäure-N-methylamid [WS-23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben), N-(4-cyanomethylphenyl)-p-menthancarboxamid, N-(2-(Pyridin-2-yl)ethyl)-3-p-menthancarboxamid, Essigsäure-2-(methylamino)-2-oxo-, 5-methyl-2-(1-methylethyl)cyclohexylester, Essigsäure-2-(ethylamino)-2-oxo-, 5-methyl-2-(1-methylethyl)cyclohexylester. Kühlwirkstoffe auf Basis eines Menthol-Grundgerüstes sind generell bevorzugte Derivate des L-Menthol. Bevorzugt ist der Einsatz eines oder mehrerer weiterer physiologischer Kühlwirkstoffe, die zwar eine physiologische Kühlwirkung verursachen, jedoch gleichzeitig keine bzw. keine relevante geschmackliche Wirkung verursachen. Bevorzugte physiologische Kühlwirkstoffe sind deshalb ausgewählt aus der Gruppe bestehend aus: Menthylether (z.B. (I-Menthoxy)-1,2-propandiol, (I-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), nicht erfindungsgemäß zu verwendende Menthancarbonsäureamide (z.B. Menthancarbonsäure-N-ethylamid [WS-3], N^{α}-(Menthancarbonyl)glycinethylester [WS-5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on), 2,2,2-Trialkylessigsäureamide (z.B. 2,2-Diisopropylpropionsäuremethylamide) oder Tetrahydropyrimidin-2-one (z.B. Icilin, Icilin-Derivate oder verwandte Verbindungen, die in WO 2004/026840 beschrieben sind).

Die Gesamtmenge an weiteren physiologischen Kühlwirkstoffen, die neben WS-12 als zusätzlicher Bestandteil in einer hierin beschriebenen Zahnputz-Zusammensetzung vorliegen, liegt vorzugsweise im Bereich von 0,01 bis 3,0 Gew.-%, bevorzugt im Bereich von 0,025 bis 1,0 Gew.-%, weiter bevorzugt im Bereich von 0,05 bis 0,50 Gew.-%, jeweils bezogen auf das Gesamtgewicht der Zahnputz-Zusammensetzung.

Eine hierin beschriebene Zahnputz-Zusammensetzung umfasst als Komponente c) ein, zwei oder mehr weitere Aromastoffe. Nicht sämtliche, zumindest aber einer dieser weiteren Aromastoffe besitzt dabei einen log-K_{ow}-Wert im Bereich von 1,5 bis 5. In manchen Fällen ist es vorteilhaft, eine Aromastoff-Mischung als Komponente c) der einzusetzenden Aromastoff-Komposition vorzusehen, in der nicht sämtliche der weiteren Aromastoffe einen log-K_{ow}-Wert im Bereich von 1,5 bis 5 besitzen.

Als Aromastoffe (mit einem log-K_{ow}-Wert im Bereich von 1,5 bis 5 oder mit einem log-K_{ow}-Wert außerhalb dieses Bereiches) eignen sich natürliche Rohstoffe wie aus Pflanzen gewonnene Extrakte und etherische Öle, bzw. daraus gewonnene Fraktionen und isolierte Stoffe, sowie auch einzelne synthetisch oder biotechnologisch gewonnene Aromastoffe.

Bevorzugte natürliche Rohstoffe sind gewählt aus der Gruppe bestehend aus: Pfefferminzöle, Krauseminzöle, Mentha-Arvensis-Öle, Anisöle, Nelkenöle, Citrusöle, Zimtrindenöle, Wintergrünöle, Cassiaöle, Davanaöle, Fichtennadelöle, Eucalyptusöle, Fenchelöle, Galbanumöle, Ingweröle, Kamillenöle, Kümmelöle, Rosenöle, Geraniumöle, Salbeiöle, Scharfgarbenöle, Sternanisöle, Thymianöle, Wacholderbeeröle, Rosmarinöle, Angelikawurzelöle, und die Fraktionen dieser Öle.

Bevorzugte Einzelverbindungen, die als weiterer Aromastoff in Komponente c) einer hierin beschriebenen Zahnputz-Zusammensetzung eingesetzt werden können, sind gewählt aus der Gruppe bestehend aus: Anethol, Menthon, Isomenthon, Menthylacetat, Menthofuran, Menthylmethylether, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis und trans Carvylacetat, p-Cymol, Thymol, 4,8-Dimethyl-3,7-nonadien-2-on, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 8-Ocimenylacetat, 2-Phenylethylalkohol, 2-Phenylethylisobutyrat, 2-Phenylethylisovalerat, Zimtaldehyd, Geraniol, Nerol. Bei chiralen Verbindungen können die genannten Aromastoffe als Racemat, als einzelnes Enantiomer oder als enantiomerenangereicherte Mischungen vorliegen. Hinsichtlich der Nachteile, die bei der Verwendung größerer Mengen von Anethol auftreten vgl. unsere obigen Ausführungen. Der Fachmann wird die Vor- und Nachteile der einzelnen Aromastoffe bzw. der einzelnen natürlichen Rohstoffe und die Fähigkeit der jeweiligen Gesamt-Aromastoff-Kompositionen zur Maskierung unangenehmer Geschmackseindrücke berücksichtigen, wenn er eine hierin beschriebene Zahnputz-Zusammensetzung formuliert.

In manchen Fällen sind hierin beschriebene Zahnputz-Zusammensetzungen bevorzugt, die in Komponente c) weitere Aromastoffe enthalten, welche einen scharfen Geschmack oder eine Wärme- oder Hitzeempfindung auf Haut und Schleimhäuten oder ein Prickel-, bzw. Kribbelgefühl im Mund- und Rachenraum hervorrufen, wie z.B. Paprikapulver, Chili-Pfeffer-Pulver, Extrakte aus Paprika, Extrakte aus Pfeffer, Extrakte aus Chili-Pfeffer, Extrakte aus Ingwerwurzeln, Extrakte aus Paradieskörnern (Aframomum melegueta), Extrakte aus Parakresse (Jambu-Oleoresin; Spilanthes acmella, bzw. Spilanthes oleracea), Extrakte aus Japanischem Pfeffer (Zanthoxylum piperitum), Extrakte aus Kaempferia galanga, Extrakte aus Alpinia galanga, Extrakte aus Wasserpfeffer (Polygonium hydropiper), Capsaicin, Dihydrocapsaicin, Gingerol, Paradol, Shogaol, Piperin, Saanshool-I, Saanshool-II, Sanshoamid, Spilanthol, Carbonsäure-N-Vanillylamide, insbesonders Nonansäure-N-vanillylamid, 2-Nonensäureamide, insbesonders 2-Nonensäure-N-isobutylamid, 2-Nonensäure-N-4-hydroxy-3-methoxyphenylamid, Alkylether von 4-Hydroxy-3-methoxybenzylalkohol, insbesonders 4-Hydroxy-3-methoxybenzyl-n-butylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, Acetale von Vanillin, Acetale von Ethylvanillin, Acetale von Isovanillin, (4-Hydroxy-3-methoxyphenyl)essigsäureamide, insbesonders (4-Hydroxy-3-methoxyphenyl)essigsäure-N-n-octylamid, Allylisothiocyanat, Nicotinaldehyd, Methylnicotinat, Propylnicotinat, 2-Butoxyethylnicotinat, Benzylnicotinat, 1-Acetoxychavicol.

In manchen Fällen bevorzugt sind hierin beschriebene Zahnputz-Zusammensetzungen, die in Komponente c) einen, zwei oder mehr weitere Aromastoffe ohne physiologische Kühlwirkung enthalten. Derartige Aromastoffe verursachen zusätzlich zu ihrem eigentlichen geruchlichen Aromawert auch einen Geschmackseindruck, einen geschmacksmodulierenden Effekt oder einen trigeminalen oder mundwässernden Reiz, nicht aber einen kühlenden Reiz. Vorzugsweise vermitteln die eingesetzten Aromastoffe einen Geschmackseindruck ausgewählt aus der Gruppe bestehend aus süß, umami, bitter, salzig und sauer. Bevorzugte geschmacksmodulierende Effekte sind ausgewählt aus der Gruppe bestehend aus bitter-maskierende, umami-verstärkende, süß-verstärkende, salzverstärkende und sauer-maskierende Effekte. Bevorzugte trigeminale Reize, die von derartigen Aromastoffen vermittelt werden, sind ausgewählt aus der Gruppe bestehend aus Schärfe, Wärme, Kribbeln und Stechen.

Bevorzugt ist der Einsatz von geschmacksmodulierenden Aromastoffen (einschließlich Geschmacksstoffen) wobei diese vorzugsweise ausgewählt sind aus der Gruppe bestehend aus Adenosin-5'-monophosphat, Cytidin-5'-monophosphat, Guanosinmonophosphat, Inosin-5'-monophosphat, Inositolphosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat, Natriumglutamat; 2-Phenoxypropionsäure; Hydroxyflavanonen, wie zum Beispiel Eriodictyol, Homoeriodictyol, und deren Natriumsalzen und Hydroxyflavanonen gemäß US 2002/0188019; Hydroxybenzoesäureamiden, wie zum Beispiel 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäu re-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N-*(4-hydroxy-3-methoxybenzyl)amid, 2,4-Dihydroxybenzoesäure-*N*-(4-hydroxy-3-methoxybenzyl)amid-Mononatriumsalz, 2,4-Dihydroxybenzoesäu re-*N*-2-(4-hydroxy-3-methoxyphenyl)ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenylalkadionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); γ-Aminobuttersäure wie beschrieben in WO 2005/096841, die bezüglich der darin offenbarten entsprechenden Verbindungen auf dem Wege der Verweisung Bestandteil dieser Anmeldung wird), Divanillin wie beschrieben in WO 2004/078302 und Hesperetin gemäß WO 2007/014879.

Bevorzugt ist auch der Einsatz von Aromastoffen ohne physiologische Kühlwirkung und dabei insbesondere von speichelflussfördernden Stoffen wie Pellitorin gemäß WO 04/000787 und US 2004/0241312 sowie Alkamiden gemäß DE 103 51 422.

Eine hierin beschriebene Zahnputz-Zusammensetzung umfasst als Komponente (i) Putzkörper. Der Begriff Putzkörper umfasst dabei insbesondere die in Zahnputz-Zusammensetzungen üblichen abrasiven Systeme, d. h. Schleif- oder Poliermittel. Die einzusetzenden Putzkörper sind dazu geeignet, Zahnbelag (Plaque) schonend, d. h. unter geringem Zahnschmelz-(Enamelum-) bzw. Zahnbein-(Dentin-) Abrieb vom Zahnschmelz zu entfernen. Vorzugsweise werden die als Komponente (i) einzusetzenden Putzkörper ausgewählt aus der Gruppe bestehend aus (vorzugsweise gefällten) Kieselsäuren, Calciumcarbonat, Calciumphosphaten, Aluminiumoxiden, Hydroxylapatiten, organischen Putzkörpern (wie z.B. Putzkörpern auf Polymethacrylat-Basis) und deren Mischungen.

Dem Fachmann ist bekannt, dass einige der vorstehend genannten weiteren Bestandteile einer bevorzugten hierin beschriebenen Zahnputz-Zusammensetzung selbst unangenehme und in manchen Fällen vor allem bittere oder metallische Geschmackseindrücke vermitteln. Überraschenderweise hat sich jedoch im Rahmen der vorliegenden Erfindung gezeigt, dass die Gesamt-Aromastoff-Komposition umfassend a) Menthol, b) eine die Bitterkeit des Menthol maskierende Menge an WS-12 sowie c) den weiteren Aromastoffen mit log-K_{ow}-Werten im Bereich von 1,5 bis 5 geeignet ist, auch diese unangenehmen (insbesondere bitteren oder metallischen) Geschmackseindrücke zu maskieren. Verantwortlich hierfür ist vermutlich die eingesetzte Menge an WS-12. Sofern die eingesetzte Menge an WS-12 die Gesamtbitterkeit der Zahnputz-Zusammensetzung maskiert, liegt für die Zwecke und nach dem Verständnis des vorliegenden Textes eine die Bitterkeit des Menthols maskierende Menge an WS-12 vor.

Insbesondere maskiert die erfindungsgemäß einzusetzende Aromastoff-Komposition mit den Komponenten a), b) und c) unangenehme, vor allem bittere oder metallische Geschmackseindrücke, wie sie durch Stoffe wie Triclosan, Zinkcitrat, -sulfat, Poly- und Pyrophosphate, Bicarbonate (z.B. Natriumbicarbonat), Strontium- und Kaliumsalze (z.B. Kaliumcitrat, Kaliumnitrat, Kaliumchlorid, Strontiumchlorid), Zinnpyrophosphat, -chlorid, Aluminiumlactat, Wasserstoffperoxid, Fluoride, Vitamine, Cetylpyridiniumchlorid sowie durch Emulgatoren, wie z.B. Natriumlaurylsulfat, Natriumlaurylsarkosinat und Cocamidopropylbetain sowie von Süßstoffen wie z.B. Aspartam, Saccharin, Acesulfam-K, Sorbit, Xylit, Cyclamate (z. B. Natriumcyclamat), Sucralose, Alitam, Neotam, Thaumatin, Neohesperidin Dihydrochalcon, Maltit, Lactit oder auch durch bestimmte Kaugummi-Massen (Gum-Basen) und deren Mischungen hervorgerufen werden.

In hierin beschriebenen Zahnputz-Zusammensetzungen kann das Gewichtsverhältnis von WS-12 zu Menthol in weiten Bereichen variieren, abhängig vom angestrebten (Maskierungs-) Ergebnis. In vielen Fällen bevorzugt sind jedoch hierin beschriebene Zahnputz-Zusammensetzungen (insbesondere vorstehend bereits als bevorzugt bezeichnete hierin beschriebene Zahnputz-Zusammensetzungen), in denen das Gewichtsverhältnis von Menthancarbonsäure-N-(4-methoxyphenyl)-amid (WS-12) zu Menthol im Bereich von 1 : 5 bis 1 : 1000 liegt, vorzugsweise im Bereich von 1 : 10 bis 1 : 500, bevorzugt im Bereich von 1 : 10 bis 1 : 250, ganz bevorzugt im Bereich von 1 : 10 bis 1 : 150.

Die vorstehend genannten bevorzugten Gewichtsverhältnisse von WS-12 zu Menthol gelten insbesondere für hierin beschriebene Zahnpasta-Zusammensetzungen und zwar insbesondere für solche Zahnpasta-Zusammensetzungen, die Pfefferminzöle enthalten.

In hierin beschriebenen Zahnputz-Zusammensetzungen kann das Gewichtsverhältnis von WS-12 zu der Gesamtmenge an weiteren Aromastoffen (Komponente c) der einzusetzenden Aromastoff-Komposition (ii)) in weiten Bereichen variieren. Bestimmte Gewichtsverhältnisse von WS-12 zu der Gesamtmenge an den weiteren Aromastoffen sind jedoch bevorzugt, insbesondere dann, wenn auch das Gewichtsverhältnis von WS-12 zu Menthol in den soeben diskutierten bevorzugten Bereichen liegt. Insbesondere bevorzugt sind hierin beschriebene Zahnputz-Zusammensetzungen, insbesondere Zahnputz-Zusammensetzungen, in denen das Gewichtsverhältnis von WS-12 zu Menthol bereits auf einen Wert im Bereich von 1 : 5 bis 1 : 1000 eingestellt ist, in denen das Gewichtsverhältnis von WS-12 zu der Gesamtmenge an den weiteren Aromastoffen mit einem log-K_{ow}-Wert im Bereich von 1,5 bis 5 höchstens 1 : 10 beträgt, vorzugsweise höchstens 1: 20 und besonders bevorzugt höchstens 1 : 40, wobei dieses Gewichtsverhältnis vorzugsweise im Bereich von 1 : 10 bis 1 : 200 liegt, bevorzugt im Bereich von 1 : 20 bis 1 : 150, besonders bevorzugt im Bereich von 1 : 40 bis 1 : 100.

Hinsichtlich des Gewichtsverhältnisses von WS-12 zu Menthol sind weiter oben gestaffelte bevorzugte Bereiche angegeben. Ebenso sind für das Gewichtsverhältnis von WS-12 zu der Gesamtmenge an weiteren Aromastoffen mit einem log-K_{ow}-Wert im Bereich von 1,5 bis 5 gestaffelte bevorzugte Bereiche angegeben. Soweit beide diskutierten Gewichtsverhältnisse in jeweils bevorzugten Bereichen liegen, ist es weiter bevorzugt, wenn zumindest eines der Gewichtsverhältnisse in einem besonders bevorzugten Bereich liegt, ganz besonders bevorzugt sind aber Kombinationen, in denen sowohl das Gewichtsverhältnis von WS-12 zu Menthol als auch das Gewichtsverhältnis von WS-12 zu den weiteren Aromastoffen mit einem log-K_{ow}-Wert in einem Bereich von 1,5 bis 5 in besonders bevorzugten Bereichen liegen.

Hinsichtlich des Gewichtsverhältnisses von WS-12 zu der Gesamtmenge der weiteren Aromastoffen mit einem log-K_{ow}-Wert im Bereich von 1,5 bis 5 ist darauf hinzuweisen, dass zur Erzielung eines langanhaltenden Kälteempfindens in einer Vielzahl von Fällen das Gewichtsverhältnis von WS-12 zu der Gesamtmenge an den weiteren Aromastoffen mit einem log-K_{ow}-Wert im Bereich von 1,5 bis 5 höchstens 1 : 10 betragen sollte. Bevorzugt ist somit, dass die besagten Aromastoffe mindestens die zehnfache Gewichtsmenge bezogen auf die Einsatzmenge von WS-12 ausmachen. Bevorzugt ist eine mindestens zwanzigfache Gewichtsmenge und besonders bevorzugt eine mindestens vierzigfache Gewichtsmenge der besagten Aromastoffe mit einem log-K_{ow}-Wert im Bereich von 1,5 bis 5.

Bevorzugt sind hierin beschriebene Zahnputz-Zusammensetzungen, in denen der Gesamtanteil an Menthancarbonsäure-N-(4-methoxyphenyl)-amid (WS-12) im Bereich von 0,00015 bis 0,26 Gew.-% liegt, vorzugsweise im Bereich von 0,0003 bis 0,13 Gew.-%, bevorzugt im Bereich von 0,0006 bis 0,13 Gew.-%, besonders bevorzugt im Bereich von 0,001 bis 0,13 Gew.-%, insbesondere bevorzugt im Bereich von 0,0016 bis 0,09 Gew.-%, ganz besonders bevorzugt im Bereich von 0,0027 bis 0,09 Gew.-%, bezogen auf die Gesamtmasse der Zahnputz-Zusammensetzung.

Diese bevorzugten Gesamtanteile an WS-12 genügen in einer Vielzahl von Fällen, um die Bitterkeit des gleichzeitig anwesenden Menthols vollständig zu neutralisieren. Bevorzugte in hierin beschriebenen Zahnputz-Zusammensetzungen einzusetzende Gesamtmengen an Menthol ergeben sich ausgehend von den bevorzugten Gesamtanteilen an Menthol unter Berücksichtigung der bevorzugten Gewichtsverhältnisse von WS-12 zu Menthol, die weiter oben diskutiert sind. Es ergibt sich daraus ein bevorzugter Gesamtanteil an Menthol in einer hierin beschriebenen Zahnputz-Zusammensetzung von 2 Gew.-% oder kleiner, bevorzugt im Bereich von 0,15 bis 1,3 Gew.-%, besonders bevorzugt im Bereich von 0,4 bis 0,9 Gew.-%.

Bei einer hierin beschriebenen Zahnputz-Zusammensetzung mit einem besonders bevorzugten Mentholanteil im Bereich von 0,4 bis 0,9 Gew.-%, bezogen auf die Gesamtmasse der Zahnputz-Zusammensetzung, liegt der Gesamtanteil an Menthancarbonsäure-N-(4-methoxyphenyl)-amid (WS-12) bevorzugt im Bereich von 0,0004 bis 0,18 Gew.-%, weiter bevorzugt im Bereich von 0,0008 bis 0,09 Gew.-%, insbesondere bevorzugt im Bereich von 0,0016 bis 0,09 Gew.-%, am meisten bevorzugt im Bereich von 0,0027 bis 0,09 Gew.-%, bezogen auf die Gesamtmasse der Zahnputz-Zusammensetzung.

Ganz besonders bevorzugt ist eine hierin beschriebene Zahnputz-Zusammensetzung umfassend:
(i) Putzkörper
(ii) eine Aromastoff-Komposition umfassend
   a) Menthol in einer Menge von 0,15 bis 1,3 Gew.-%, vorzugsweise in einer Menge von 0,4 bis 0,9 Gew.-%,
   b) eine die Bitterkeit des Menthol maskierende Menge an Menthancarbonsäure-N-(4-methoxyphenyl)-amid (WS-12) im Bereich von 0,00015 bis 0,26 Gew.-%, wobei das Gewichtsverhältnis von Menthancarbonsäure-N-(4-methoxyphenyl)-amid (WS-12) zu Menthol im Bereich von 1: 5 bis 1 : 1000 liegt, sowie
   c) einen, zwei oder mehr weitere Aromastoffe, wobei der, mehrere oder sämtliche der weiteren Aromastoffe einen log-K_{ow}-Wert im Bereich von 1,5 bis 5 besitzen und wobei das Gewichtsverhältnis von Menthancarbonsäure-N-(4-methoxyphenyl)-amid (WS-12) zu der Gesamtmenge an den weiteren Aromastoffen mit einem log-K_{ow}-Wert im Bereich von 1,5 bis 5 höchstens 1 : 10 beträgt,
   wobei die Gew.-%-Angaben bezogen sind auf das Gesamtgewicht der Zahnputz-Zusammensetzung.

Hierin beschrieben ist auch die Verwendung von
- Menthancarbonsäure-N-(4-methoxyphenyl)-amid (WS-12) zum Maskieren des bitteren Geschmackes von Menthol in einer Zahnputz-Zusammensetzung,
   wobei das Menthancarbonsäure-N-(4-methoxyphenyl)-amid (WS-12) zum Zwecke des Maskierens in Mischung mit
- einem, zwei oder mehr weiteren Aromastoffen vorliegt oder zu bringen ist, wobei der, mehrere oder sämtliche der weiteren Aromastoffe einen log-K_{ow}-Wert im Bereich von 1,5 bis 5 besitzen.

Es versteht sich, dass hinsichtlich dieser Verwendung die vorstehend mit Blick auf die hierin beschriebenen Zahnputz-Zusammensetzung diskutierten bevorzugten Ausgestaltungen entsprechend gelten.

So ist insbesondere eine solche Verwendung bevorzugt, wobei der, mehrere oder sämtliche der weiteren Aromastoffe ausgewählt sind aus der Gruppe bestehend aus Menthon, Isomenthon, Carvon, 1,2-Dihydrocarvon, Anethol, Piperiton, Menthylacetat, Menthylmethylether, 1,8-Cineol, Zimtaldehyd und Methylsalicylat, vorzugsweise aus der Gruppe bestehend aus Menthon, Isomenthon, Carvon und Piperiton.

Ebenfalls bevorzugt ist eine solche Verwendung (insbesondere die vorstehend als bevorzugt bezeichnete Verwendung), wobei in der Mischung das Gewichtsverhältnis von Menthancarbonsäure-N-(4-methoxyphenyl)-amid (WS-12) zu der Gesamtmenge an den weiteren Aromastoffen mit einem log-K_{ow}-Wert im Bereich von 1,5 bis 5 im Bereich von 1 : 10 bis 1 : 200 liegt, vorzugsweise im Bereich von 1 : 20 bis 1 : 150, besonders bevorzugt im Bereich von 1 : 40 bis 1 : 100.

Hierin beschrieben ist auch ein Verfahren zum Lösen von Menthancarbonsäure-N-(4-methoxyphenyl)-amid (WS-12) in einer Zahnputz-Zusammensetzung umfassend Menthol, wobei
- Menthol, vorzugsweise 5 bis 1000 Gewichtsteile Menthol, bevorzugt 10 bis 500 Gewichtsteile Menthol, besonders bevorzugt 10 bis 250 Gewichtsteile, ganz besonders bevorzugt 10 bis 150 Gewichtsteile Menthol,
- 1 Gewichtsteil Menthancarbonsäure-N-(4-methoxyphenyl)-amid (WS-12) und
- insgesamt 10 bis 200, vorzugsweise 20 bis 150, besonders bevorzugt 40 bis 100 Gewichtsteile an einem, zwei oder mehr weiteren Aromastoffen, wobei der, mehrere oder sämtliche der weiteren Aromastoffe einen log-K_{ow}-Wert im Bereich von 1,5 bis 5 besitzen,
   sowie
- weitere Zahnputz-Komponenten vermischt werden.

Dieser Aspekt geht auf die überraschende Erkenntnis zurück, dass das im Rahmen der vorliegenden Erfindung als Maskierungsmittel eingesetzte WS-12 in einer Zahnputz-Zusammensetzung umfassend Menthol nur dann in ausreichenden Mengen in Lösung gebracht werden kann, wenn Substanzen mit log-K_{ow}-Wert im Bereich von 1,5 bis 5 vorliegen. Im Rahmen des vorliegenden Textes handelt es sich bei diesen Substanzen mit einem log-K_{ow}-Wert im Bereich von 1,5 bis 5 um weitere Aromastoffe, sodass sich neben dem für die Löslichkeit entscheidenden Aspekt auch weitere positive Eigenschaften einer resultierenden Zahnputz-Zusammensetzung hinsichtlich der vermittelten Geschmackseindrücke ergeben.

Hierin beschrieben ist auch ein Verfahren zum Maskieren der Bitterkeit von Menthol in einer Zahnputz-Zusammensetzung mit folgendem Schritt:
Mischen von
a) Menthol mit
b) einer die Bitterkeit des Menthol maskierenden Menge an Menthancarbonsäure-N-(4-methoxyphenyl)-amid (WS-12),
c) einem, zwei oder mehr weiteren Aromastoffen, wobei der, mehrere oder sämtliche der weitere Aromastoffe einen log-K_{ow}-Wert im Bereich von 1,5 bis 5 besitzen, und die eingesetzte Menge an diesen weiteren Aromastoffen so gewählt ist, dass die Löslichkeit des Menthancarbonsäure-N-(4-methoxyphenyl)-amids (WS-12) erhöht wird, sowie
d) weiteren Zahnputz-Komponenten.

Es versteht sich, dass hinsichtlich dieses Verfahrens die vorstehend mit Blick auf die hierin beschriebene Zahnputz-Zusammensetzung diskutierten bevorzugten Ausgestaltungen entsprechend gelten.

Im Folgenden werden die hierin beschriebenen Aspekte an Hand von Beispielen näher erläutert.

Sofern nicht anders angegeben beziehen sich dabei alle (Mengen-) Angaben auf das Gewicht.

### Beispiele:

In den folgenden Beispielen werden verschiedene ätherische Öle verwendet. Für die Berechnung des Gesamtanteils von Menthol sowie des Anteils der ein, zwei oder mehr weiteren Aromastoffe mit einem log-K_{ow}-Wert in einem Bereich von 1,5 bis 5 in der jeweiligen Aromastoff-Komposition (ii) werden folgende Anteile in den verwendeten ätherischen Ölen zugrunde gelegt:
Pfefferminzöl Mentha arvensis, rektifiziert (handelsübliche dementholisierte Qualität): 1,8-Cineol (Eucalyptol) ca. 0,4%; Menthon ca. 35%; Menthol ca. 40%.

Pfefferminzöl Mentha piperita Typ Willamette: 1,8-Cineol (Eucalyptol) ca. 5%; Menthon ca. 30%; Menthol ca. 40%.

Krauseminzöl Typ Native: 1,8-Cineol (Eucalyptol) ca. 2,2%; Carvon ca. 70%.

Krauseminzöl Typ Midwest Scotch: 1,8-Cineol (Eucalyptol) ca. 1,5%; Carvon ca.70%.

In den folgenden Beispielen wird bei der Benutzung von Krauseminzöl der Typ Native verwendet. An Stelle des Krauseminzöls Typ Native ist auch die Benutzung des Typ Midwest Scotch vorteilhaft. Die Gehaltsberechnungen der betreffenden Beispiele mit Krauseminzöl beziehen sich auf die Gehaltsangaben zu Krauseminzöl Typ Native.

In einigen Beispielen wurde eine Pellitorin-Lösung (im Folgenden als "Pellitorin Lösung PLM" bezeichnet) eingesetzt, bestehend aus 10% Pellitorin (umfassend 4,9% 2E,4Z-Decadiensäure-N-isobutylamid und 94,3% 2E,4E-Decadiensäure-N-isobutylamid sowie 0,8% Lösungsmittel), 45% Propylenglycol und 45% natürlichem Pfefferminzöl (Mentha arvensis, rektifiziert).

### Beispiel 1:

### Zahnputz-Zusammensetzung umfassend eine Aromastoff-Komposition vom Eucalyptus-Menthol-Typ - vergleichende Untersuchungen

### Beispiel 1.1: Reduzierung der Bitternote von Menthol in Zahnputz-Zusammensetzungen durch einen Anteil an Anethol (Vergleichsbeispiel)

### 1. Ansatz:

Es wurden vermischt:

| | | |
|---|---|---|
| 20 | Gew.-% | Pfefferminzöl Mentha piperita Typ Willamette |
| 20 | Gew.-% | Pfefferminzöl Mentha arvensis, rektifiziert |
| 1 | Gew.-% | *I*-Menthylacetat |
| 2 | Gew.-% | 2-Hydroxyethylmenthylcarbonat |
| 2 | Gew.-% | 2-Hydroxypropylmenthylcarbonat |
| 5 | Gew.-% | 1,8-Cineol (Eucalyptol) |
| 40 | Gew.-% | *I*-Menthol |
| 10 | Gew.-% | Anethol |

### 2. Ansatz:

In einem 2. Ansatz wurden die Bestandteile des 1. Ansatzes in den gleichen Verhältnissen zueinander, jedoch ohne Anethol, gemischt.

Die so erhaltenen Aromastoff-Kompositionen wurden jeweils in eine jeweilige Standard-Zahnpasta-Masse auf Kieselsäure-Basis in einer Konzentration von 1,2 Gew.-% eingearbeitet, bezogen auf die Gesamtmasse der resultierenden Zahnpasta. Die Zahnpasten wurden von einem sensorisch geschulten Expertenpanel unter Praxisbedingungen getestet. Die sensorische Bewertung ergab, dass trotz des hohen Gehaltes an Menthol in der Aromastoff-Komposition (56 Gew.-% Gesamtmentholgehalt, davon 40 Gew.-% linear zugesetztes Menthol) durch den Einsatz von 10 Gew.-% Anethol im 1. Ansatz die bitter-scharfe Note des Menthols neutralisiert und somit insgesamt ein angenehmes Geschmacksergebnis erzielt wurde. Ohne den Einsatz von Anethol im 2. Ansatz tritt die bitter-scharfe Note deutlich hervor und das Geschmacksergebnis wird als insgesamt nicht akzeptabel empfunden.

### Beispiel 1.2: Reduzierung der Bitternote von Menthol in Zahnputz-Zusammensetzungen durch einen Anteil an Menthancarbonsäure-N-(4-methoxyphenyl)-amid (WS-12)

### 1. Ansatz

Es wurden vermischt:

| | | |
|---|---|---|
| 25 | Gew.-% | Pfefferminzöl Mentha piperita Typ Willamette |
| 25 | Gew.-% | Pfefferminzöl Mentha arvensis, rektifiziert |
| 1 | Gew.-% | *I*-Menthylacetat |
| 2 | Gew.-% | 2-Hydroxyethylmenthylcarbonat |
| 2 | Gew.-% | 2-Hydroxypropylmenthylcarbonat |
| 5 | Gew.-% | 1,8-Cineol (Eucalyptol) |
| 39,5 | Gew.-% | *I*-Menthol |
| 0,5 | Gew.-% | Menthancarbonsäure-N-(4-methoxyphenyl)-amid |

### 2. Ansatz:

In einem 2. Ansatz wurden die Bestandteile des 1. Ansatzes in den gleichen Verhältnissen zueinander, jedoch ohne Menthancarbonsäure-N-(4-methoxyphenyl)-amid (WS-12), gemischt.

Die so erhaltenen Aromastoff-Kompositionen wurden jeweils in eine jeweilige Standard-Zahnpasta-Masse auf Kieselsäure-Basis in einer Konzentration von 1,2 Gew.-% eingearbeitet, bezogen auf die Gesamtmasse der resultierenden Zahnpasta. Die Zahnpasten wurden von einem sensorisch geschulten Expertenpanel unter Praxisbedingungen getestet. Die sensorische Bewertung ergab, dass bei Einsatz von 0,5 Gew.-% Menthancarbonsäure-N-(4-methoxyphenyl)-amid (WS-12) im 1. Ansatz die bitter-scharfe Note des Menthols (im Vergleich zu dem 2. Ansatz) deutlich reduziert wurde und die Zahnpasta aromatisch als voller und runder empfunden wurde. Die Aromastoff-Komposition bzw. die Zahnpasta zeigte eine sehr schöne, reiche minzige Frische mit einem sehr starken und ausgeprägten, sehr lang anhaltenden kühlenden Frischeempfinden.

Ohne den Einsatz von Menthancarbonsäure-N-(4-methoxyphenyl)-amid (WS-12) trat die bitter-scharfe Note des Menthols deutlich hervor, und das Aroma wurde als wesentlich weniger angenehm empfunden. Der Eindruck eines kühlenden Frischeempfindens hielt auch signifikant weniger lange an.

In der oben beschriebenen Aromastoff-Komposition gemäß 1. Ansatz (Beispiel 2.2) beträgt der Anteil an linear zugesetztem Menthol 40 Gew.-% (bei einem Gesamtmentholgehalt - durch das zudem in den Pfefferminzölen enthaltene Menthol - von 59,5 Gew.-%). Das Gewichtsverhältnis von Menthancarbonsäure-N-(4-methoxyphenyl)-amid (WS-12) zu linear zugesetztem Menthol beträgt damit 1 : 80. Das Gewichtsverhältnis von Menthancarbonsäure-N-(4-methoxyphenyl)-amid (WS-12) zur Gesamtmenge an Menthol beträgt 1 : 119. Das Gewichtsverhältnis von Menthancarbonsäure-N-(4-methoxyphenyl)-amid (WS-12) zu der Gesamtmenge an weiteren Aromastoffen mit einem log-K_{ow}-Wert im Bereich von 1,5 bis 5 (I-Menthylacetat, 1,8-Cineol, sowie oben erwähnte entsprechende Anteile der Pfefferminzöle) beträgt 1 : 47.

### Beispiel 2:

### Verlängerung des durch eine Zahnputz-Zusammensetzung bewirkten kühlenden Frischeempfindens durch Verwendung von Menthancarbonsäure-N-(4-methoxyphenyl)-amid (WS-12)

Der Anteil an Menthancarbonsäure-N-(4-methoxyphenyl)-amids (WS-12) im 1. Ansatz von Beispiel 1.2 wurde durch Menthan-3-carbonsäure-N-ethylamid (WS-3) ersetzt.

Die erhaltene Aromastoff-Komposition wurde ebenfalls in eine Standard-Zahnpasta-Masse auf Kieselsäure-Basis in einer Konzentration von 1,2 Gew.-% eingearbeitet, bezogen auf die Gesamtmasse der resultierenden Zahnpasta.

Die so bereitete Zahnpasta wurde von einem sensorisch geschulten Expertenpanel unter Praxisbedingungen getestet und mit der Menthancarbonsäure-N-(4-methoxyphenyl)-amid (WS-12) enthaltenden Zahnpasta gemäß 1. Ansatz von Beispiel 1.2 in Bezug auf die Bitterwahrnehmung und die Dauer der kühlenden Frischeempfindung verglichen.

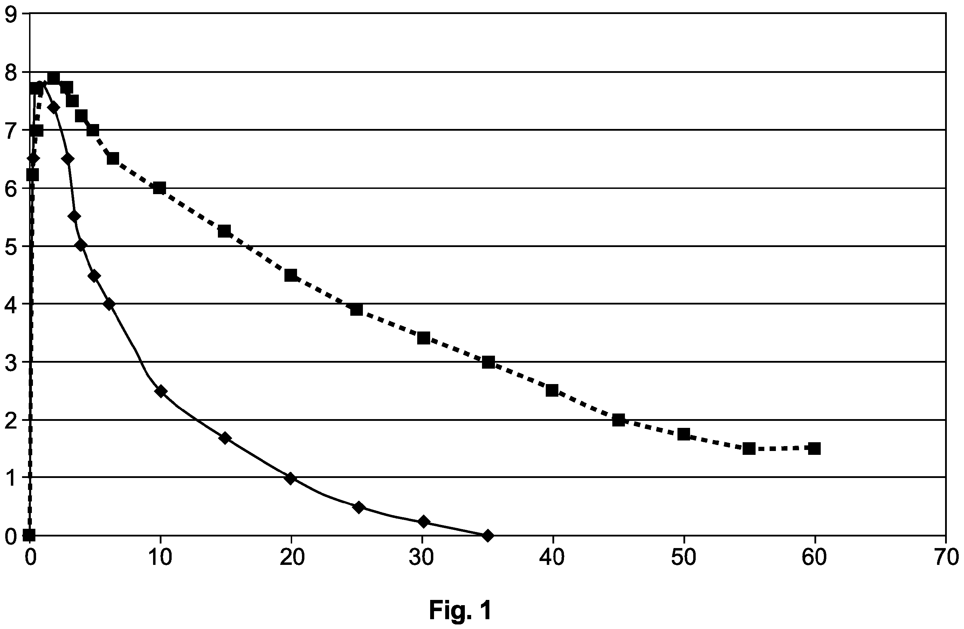

Bei der Zahnpasta mit der Aromastoff-Komposition, die Menthan-3-carbonsäure-N-ethylamid (WS-3) enthält, wurde die Bitterwahrnehmung kaum reduziert, wogegen bei der Zahnpasta, umfassend die Aromastoff-Komposition mit Menthancarbonsäure-N-(4-methoxyphenyl)-amid (WS-12), die Bitterwahrnehmung deutlich reduziert worden ist. Zudem wurde die Zahnpasta mit Menthancarbonsäure-N-(4-methoxyphenyl)-amid (WS-12) als aromatisch harmonischer, runder und voller empfunden. Unterstützt wurde dieser Eindruck durch das vergleichsweise langanhaltende Frischeempfinden, das durch die Verwendung von Menthancarbonsäure-N-(4-methoxyphenyl)-amid (WS-12) ausgelöst wurde. Der zeitliche Verlauf der kühlenden Frischeempfindung ist in Fig.1 illustriert.

Fig.1 zeigt die Intensität und Dauer der kühlenden Frischeempfindung von Zahnpasten enthaltend eine Aromastoff-Komposition gemäß a) Beispiel 1.2 (1. Ansatz) mit Menthan-3-carbonsäure-N-ethylamid (WS-3) - dargestellt mit einer durchgezogenen Linie mit Rautensymbolen - oder gemäß b) Beispiel 1.2 (1. Ansatz) mit Menthan-3-carbonsäure-N-(4-methoxyphenyl)-amid (WS-12) - dargestellt mit einer gestrichelten Linie mit Quadraten. Die Abszisse von Fig.1 gibt die Zeit in Minuten an, die seit dem Zähneputzen mit der jeweiligen Zahnpasta vergangen ist.

Die Ordinate von Fig.1 gibt die wahrgenommene Kühlintensität an. Die wahrgenommene Kühlintensität wurde auf einer Skala von 0 bis 10 eingeordnet, wobei der Wert "0" der am geringsten bzw. keiner wahrgenommenen Kühlintensität und der Wert "8" der am höchsten wahrgenommenen Kühlintensität entsprach.

Aus Fig.1 ist unmittelbar erkennbar, dass die Zahnpasta enthaltend eine Aromastoff-Komposition mit Menthan-3-carbonsäure-N-(4-methoxyphenyl)-amid (WS-12) im Vergleich zu der Zahnpasta enthaltend eine Aromastoff-Komposition mit Menthan-3-carbonsäure-N-ethylamid (WS-3) ein deutlich längeres Frischeempfinden vermittelt. Nach 35 Minuten liegt bei der Zahnpasta enthaltend eine Aromastoff-Komposition mit Menthan-3-carbonsäure-N-ethylamid (WS-3) eine mit dem Wert "0" wahrgenommene sehr geringe bzw. keine Kühlintensität mehr vor, wohingegen jedoch bei der Zahnpasta enthaltend eine Aromastoff-Komposition mit Menthan-3-carbonsäure-N-(4-methoxyphenyl)-amid (WS-12) nach 35 Minuten noch eine Kühlintensität mit einem Wert von "3" wahrgenommen wird, die auch nach 60 Minuten noch bei einem Wert zwischen "1" und "2" (bei ca. "1,5") liegt.

### Beispiel 3:

### Nicht bzw. nur geringfügig bitter schmeckende Zahnpasta umfassend eine Aromastoff-Komposition mit hohem Menthol-Gehalt und Zimtgeschmack

Zur Herstellung einer Aromastoff-Komposition wurden vermischt:

| | | |
|---|---|---|
| 5 | Gew.-% | Zimtaldehyd |
| 22,5 | Gew.-% | Pfefferminzöl Mentha piperita Typ Willamette |
| 22,5 | Gew.-% | Pfefferminzöl Mentha arvensis, rektifiziert |
| 1 | Gew.-% | *I*-Menthylacetat |
| 2 | Gew.-% | 2-Hydroxyethylmenthylcarbonat |
| 2 | Gew.-% | 2-Hydroxypropylmenthylcarbonat |
| 5 | Gew.-% | 1,8-Cineol (Eucalyptol) |
| 39,5 | Gew.-% | *I*-Menthol |
| 0,5 | Gew.-% | Menthancarbonsäure-N-(4-methoxyphenyl)-amid. |

Die so erhaltene Aromastoff-Komposition wurde in eine Standard-Zahnpasta-Masse auf Kieselsäure-Basis in einer Konzentration von 1,2 Gew.-% eingearbeitet, bezogen auf die Gesamtmasse der resultierenden Zahnpasta. Die Zahnpasta wurde von einem sensorisch geschulten Expertenpanel unter Praxisbedingungen getestet. Die sensorische Bewertung ergab, dass trotz des hohen Gehaltes an Menthol die Bitternote stark reduziert war. Da durch den Einsatz von Menthancarbonsäure-N-(4-methoxyphenyl)-amid (WS-12) in Kombination mit den weiteren Aromastoffen mit einem log-K_{ow}-Wert im Bereich von 1,5 bis 5 (I-Menthylacetat, 1,8-Cineol, Zimtaldehyd, sowie oben erwähnte entsprechende Anteile der Pfefferminzöle) eine starke Bitterreduktion (des Menthols) erzielt wurde, konnte vorteilhafterweise auf den Einsatz von Anethol zur Bitterreduktion verzichtet werden. Neben einem angenehmen, deutlich frischen, lang anhaltenden Geschmackseindruck konnte der Zahnpasta durch den Anteil an Zimtaldehyd eine deutliche, süße Zimtnote verliehen werden.

In der obigen Aromastoff-Komposition beträgt der Anteil an linear zugesetztem Menthol 39,5 Gew.-% (bei einem Gesamtmentholgehalt - durch das zudem in den Pfefferminzölen enthaltene Menthol - von 57,5 Gew.-%). Das Gewichtsverhältnis von Menthancarbonsäure-N-(4-methoxyphenyl)-amid (WS-12) zu linear zugesetztem Menthol beträgt damit 1 : 79. Das Gewichtsverhältnis von Menthancarbonsäure-N-(4-methoxyphenyl)-amid (WS-12) zur Gesamtmenge an Menthol beträgt 1 : 115. Das Gewichtsverhältnis von Menthancarbonsäure-N-(4-methoxyphenyl)-amid (WS-12) zu der Gesamtmenge an den weiteren Aromastoffen mit einem log-K_{ow}-Wert im Bereich von 1,5 bis 5 (I-Menthylacetat, 1,8-Cineol, Zimtaldehyd, sowie oben erwähnte entsprechende Anteile der Pfefferminzöle) beträgt 1 : 54.

### Beispiel 4:

### Zahnputz-Zusammensetzung umfassend eine Aromastoff-Komposition mit Krauseminz-Charakter - vergleichende Untersuchungen

### Beispiel 4.1 (Vergleichsbeispiel):

Durch Vermischen von

| | | |
|---|---|---|
| 30 | Gew.-% | Menthol |
| 20 | Gew.-% | Carvon |
| 20 | Gew.-% | Krauseminzöl Typ Native |
| 5 | Gew.-% | Anethol |
| 10 | Gew.-% | Pfefferminzöl Mentha arvensis, rektifiziert |
| 15 | Gew.-% | Pfefferminzöl Mentha piperita Typ Willamette |

wurde eine Aromastoff-Komposition für Zahnputz-Zusammensetzungen mit Krauseminz-Charakter hergestellt.

### Beispiel 4.2:

Durch Vermischen von

| | | |
|---|---|---|
| 39,5 | Gew.-% | Menthol |
| 20 | Gew.-% | Carvon |
| 20 | Gew.-% | Krauseminzöl Typ Native |
| 10 | Gew.-% | Pfefferminzöl Mentha arvensis, rektifiziert |
| 10 | Gew.-% | Pfefferminzöl Mentha piperita Typ Willamette |
| 0,5 | Gew.-% | Menthancarbonsäure-N-(4-methoxyphenyl)-amid |

wurde eine Aromastoff-Komposition mit einem Anteil von 0,5 Gew.-% Menthancarbonsäure-N-(4-methoxyphenyl)-amid (WS-12) für Zahnputz-Zusammensetzungen mit Krauseminz-Charakter hergestellt.

Die Aromastoff-Kompositionen gemäß Beispiel 4.1 (für eine Vergleichs-Zahnputz-Zusammensetzung) und Beispiel 4.2 (für eine hierin beschriebene Zahnputz-Zusammensetzung) wurden jeweils mit einer Konzentration von 1,2 Gew.-%, bezogen auf die Gesamtmasse der resultierenden Zahnpasta, in eine jeweilige Zahnpasta-Masse eingearbeitet, die zu einem Anteil von 65 Gew.-% aus Natriumbicarbonat bestand.

Die daraus resultierenden Zahnpasten wurden unter Praxisbedingungen getestet und von einem sensorisch geschulten Expertenpanel bewertet. Die sensorische Bewertung für die (hierin beschriebene) Zahnpasta enthaltend eine Aromastoff-Komposition gemäß Beispiel 4.2 ergab einen sehr kräftigen, angenehmen, frisch-minzigen Krauseminzgeschmack verbunden mit einem lang anhaltenden Frischeempfinden. Im Vergleich zu der Zahnpasta mit der Aromastoff-Komposition gemäß Beispiel 4.1, d.h. ohne Menthancarbonsäure-N-(4-methoxyphenyl)-amid (WS-12), aber mit Anethol, wurde das Frischeempfinden bei der Zahnpasta enthaltend eine Aromastoff-Komposition gemäß Beispiel 4.2 erheblich verstärkt und dadurch der typische Krauseminz-Charakter deutlich betont. Durch den Anteil an 0,5 Gew.-% an Menthancarbonsäure-N-(4-methoxyphenyl)-amid (WS-12) in der Aromastoff-Komposition gemäß Beispiel 4.2 wurde der zur Bitterreduktion in Beispiel 4.1 eingesetzte Anteil an 5 Gew.-% Anethol nunmehr nicht mehr für die Bitterreduktion des Menthols benötigt. Die eingesetzte Menge an Menthancarbonsäure-N-(4-methoxyphenyl)-amid (WS-12) war für die Zwecke der Bitterreduktion ausreichend. Zudem konnte der linear zugesetzte Menthol-Anteil in der Aromastoff-Komposition vorteilhafterweise von 30 Gew.-% (Beispiel 4.1) auf 39,5 Gew.-% (Beispiel 4.2) erhöht werden, ohne dass sich eine bitter-scharfe Note des Menthols einstellte, wie es bei einer Aromastoff-Komposition gemäß Beispiel 4.1 bei einem derart erhöhten Anteil an linear zugesetztem Menthol erwartet worden wäre.

In der oben beschriebenen Aromastoff-Komposition gemäß Beispiel 4.2 beträgt der Anteil an linear zugesetztem Menthol 39,5 Gew.-% (bei einem Gesamtmentholgehalt - durch das zudem in den Pfefferminzölen enthaltene Menthol - von 47,5 Gew.-%). Das Gewichtsverhältnis von Menthancarbonsäure-N-(4-methoxyphenyl)-amid (WS-12) zu linear zugesetztem Menthol beträgt damit 1 : 79. Das Gewichtsverhältnis von Menthancarbonsäure-N-(4-methoxyphenyl)-amid (WS-12) zur Gesamtmenge an Menthol beträgt 1 : 95. Das Gewichtsverhältnis von Menthancarbonsäure-N-(4-methoxyphenyl)-amid (WS-12) zu der Gesamtmenge an weiteren Aromastoffen mit einem log-K_{ow}-Wert im Bereich von 1,5 bis 5 (Carvon, sowie oben erwähnte entsprechende Anteile der Pfefferminzöle und des Krauseminzöles) beträgt 1 : 83.

### Beispiel 5:

Zahnputz-Zusammensetzung umfassend eine Aromastoff-Komposition mit würzigaromatischer Note - vergleichende Untersuchungen.

### Beispiel 5.1 (Vergleichsbeispiel):

Durch Vermischen von

| | | |
|---|---|---|
| 30 | Gew.-% | *I*-Menthol |
| 25 | Gew.-% | Pfefferminzöl Mentha arvensis, rektifiziert |
| 15 | Gew.-% | Pfefferminzöl Mentha piperita Typ Willamette |
| 10 | Gew.-% | Anethol |
| 10 | Gew.-% | Krauseminzöl Typ Native |
| 5 | Gew.-% | Zimtaldehyd |
| 5 | Gew.-% | Eugenol |

wurde eine Aromastoff-Komposition für Zahnputz-Zusammensetzungen mit einer würzig-aromatischen Note hergestellt.

### Beispiel 5.2:

Durch Vermischen von

| | | |
|---|---|---|
| 40 | Gew.-% | *I*-Menthol |
| 22 | Gew.-% | Pfefferminzöl Mentha arvensis, rektifiziert |
| 12,5 | Gew.-% | Pfefferminzöl Mentha piperita Typ Willamette |
| 5 | Gew.-% | Anethol |
| 10 | Gew.-% | Krauseminzöl Typ Native |
| 5 | Gew.-% | Zimtaldehyd |
| 5 | Gew.-% | Eugenol |
| 0,5 | Gew.-% | Menthancarbonsäure-N-(4-methoxyphenyl)-amid |

wurde eine Aromastoff-Komposition mit einem Anteil von 0,5 Gew.-% Menthancarbonsäure-N-(4-methoxyphenyl)-amid (WS-12) für Zahnputz-Zusammensetzungen mit einer würzig-aromatischen Note hergestellt.

Die Aromastoff-Kompositionen gemäß Beispiel 5.1 (für eine Vergleichs-Zahnputz-Zusammensetzung) und Beispiel 5.2 (für eine hierin beschriebene Zahnputz-Zusammensetzung) wurden jeweils mit einer Konzentration von 1,2 Gew.-% in eine jeweilige Standard-Zahnpasta-Masse auf Kieselsäure-Basis eingearbeitet, bezogen auf die Gesamtmasse der resultierenden Zahnpasta.

Die daraus resultierenden Zahnpasten wurden von einem sensorisch geschulten Expertenpanel unter Praxisbedingungen getestet. Die sensorische Bewertung für die (hierin beschriebene) Zahnpasta enthaltend eine Aromastoff-Komposition gemäß Beispiel 5.2 ergab eine betont minzige frisch-aromatische Geschmacksnote mit einem sehr starken und ausgeprägten, sehr lang anhaltenden, kühlenden Frischeempfinden.

Durch den Anteil an 0,5 Gew.-% Menthancarbonsäure-N-(4-methoxyphenyl)-amid (WS-12) in der Aromastoff-Komposition gemäß Beispiel 5.2 konnte der zur Bitterreduktion in Beispiel 5.1 eingesetzte Anethol-Anteil von 10 Gew.-% auf nunmehr 5 Gew.-% reduziert werden. Zudem konnte der linear zugesetzte Menthol-Anteil in der Aromastoff-Komposition von 30 Gew.-% (Beispiel 5.1) auf 40 Gew.-% (Beispiel 5.2) erhöht werden, ohne dass sich eine bitter-scharfe Note des Menthols einstellte, wie es bei einer Aromastoff-Komposition gemäß Beispiel 5.1 bei einem derart erhöhten Anteil an linear zugesetztem Menthol erwartet worden wäre.

In der oben beschriebenen Aromastoff-Komposition gemäß Beispiel 5.2 beträgt der Anteil an linear zugesetztem Menthol 40 Gew.-% (bei einem Gesamtmentholgehalt - durch das zudem in den Pfefferminzölen enthaltene Menthol - von 53,8 Gew.-%). Das Gewichtsverhältnis von Menthancarbonsäure-N-(4-methoxyphenyl)-amid (WS-12) zu linear zugesetztem Menthol beträgt damit 1 : 80. Das Gewichtsverhältnis von Menthancarbonsäure-N-(4-methoxyphenyl)-amid (WS-12) zur Gesamtmenge an Menthol beträgt 1 : 108.

Das Gewichtsverhältnis von Menthancarbonsäure-N-(4-methoxyphenyl)-amid (WS-12) zu der Gesamtmenge an weiteren Aromastoffen mit einem log-K_{ow}-Wert im Bereich von 1,5 bis 5 (Anethol, Zimtaldehyd, sowie oben erwähnte entsprechende Anteile der Pfefferminzöle und des Krauseminzöles) beträgt 1 : 59.

### Beispiel 6:

### Zahnputz-Zusammensetzung umfassend eine Aromastoff-Komposition mit Wintergrün-Charakter - vergleichende Untersuchungen

### Beispiel 6.1 (Vergleichsbeispiel):

Durch Vermischen von

| | | |
|---|---|---|
| 10 | Gew.-% | Anethol |
| 12,5 | Gew.-% | Pfefferminzöl Mentha arvensis, rektifiziert |
| 12,5 | Gew.-% | Pfefferminzöl Mentha piperita Typ Willamette |
| 25 | Gew.-% | Methylsalicylat |
| 40 | Gew.-% | *I*-Menthol |

wurde eine Aromastoff-Komposition für Zahnputz-Zusammensetzungen mit Wintergrün-Charakter hergestellt.

### Beispiel 6.2:

Durch Vermischen von:

| | | |
|---|---|---|
| 17 | Gew.-% | Pfefferminzöl Mentha arvensis, rektifiziert |
| 17,5 | Gew.-% | Pfefferminzöl Mentha piperita Typ Willamette |
| 25 | Gew.-% | Methylsalicylat |
| 40 | Gew.-% | *I*-Menthol |
| 0,5 | Gew.-% | Menthancarbonsäure-N-(4-methoxyphenyl)-amid |

wurde eine Aromastoff-Komposition mit einem Anteil von 0,5 Gew.-% Menthancarbonsäure-N-(4-methoxyphenyl)-amid (WS-12) für Zahnputz-Zusammensetzungen mit Wintergrün-Charakter hergestellt.

Die Aromastoff-Kompositionen gemäß Beispiel 6.1 (für eine Vergleichs-Zahnputz-Zusammensetzung) und Beispiel 6.2 (für eine hierin beschriebene Zahnputz-Zusammensetzung) wurden jeweils mit einer Konzentration von 1,2 Gew.-% in eine jeweilige Standard-Zahnpasta-Masse auf Kieselsäure-Basis eingearbeitet, bezogen auf die Gesamtmasse der resultierenden Zahnpasta.

Die daraus resultierenden Zahnpasten wurden von einem sensorisch geschulten Expertenpanel unter Praxisbedingungen getestet. Die sensorische Bewertung für die Zahnpasta enthaltend eine Aromastoff-Komposition gemäß Beispiel 6.2 ergab eine ausgeprägte frisch-pfefferminzige Wintergrün-Note mit einem sehr starken und ausgeprägten, sehr lang anhaltenden, kühlenden Frischeempfinden. Durch den Anteil von 0,5 Gew.-% Menthancarbonsäure-N-(4-methoxyphenyl)-amid (WS-12) in der Aromastoff-Komposition gemäß Beispiel 6.2 wurde der zur Bitterreduktion in Beispiel 6.1 eingesetzte Anteil an Anethol (10 Gew.-%) nicht mehr für die Bitterreduktion des Menthols benötigt. Zudem konnte vorteilhafterweise der (Gesamt-)Menthol-Anteil in der Aromastoff-Komposition durch die Erhöhung der Gew.-%-Anteile des Pfefferminzöles Mentha arvensis, rektifiziert, von 12,5 Gew.-% (Beispiel 6.1) auf 17 Gew.-% (Beispiel 6.2) und des Pfefferminzöles Mentha piperita Typ Willamette von 12,5 Gew.-% (Beispiel 6.1) auf 17,5 Gew.-% (Beispiel 6.2) erhöht werden, ohne dass sich die bitter-scharfe Note des Menthols einstellte.

In der oben beschriebenen Aromastoff-Komposition gemäß Beispiel 6.2. beträgt der Anteil an linear zugesetztem Menthol 40 Gew.-% (bei einem Gesamtmentholgehalt - durch das zudem in den Pfefferminzölen enthaltene Menthol - von 54 Gew.-%). Das Gewichtsverhältnis von Menthancarbonsäure-N-(4-methoxyphenyl)-amid (WS-12) zu linear zugesetztem Menthol beträgt damit 1 : 80. Das Gewichtsverhältnis von Menthancarbonsäure-N-(4-methoxyphenyl)-amid (WS-12) zur Gesamtmenge an Menthol beträgt 1 : 108.

Das Gewichtsverhältnis von Menthancarbonsäure-N-(4-methoxyphenyl)-amid (WS-12) zu der Gesamtmenge an weiteren Aromastoffen mit einem log-K_{ow}-Wert im Bereich von 1,5 bis 5 (Methylsalicylat, sowie oben erwähnte entsprechende Anteile der Pfefferminzöle) beträgt 1 : 74.

### Beispiel 7:

Mundwasser bzw. Mundwasserkonzentrat umfassend eine Aromastoff-Komposition mit Eucalyptus-Charakter - vergleichende Untersuchungen

### Beispiel 7.1 (Vergleichsbeispiel):

Durch Vermischen von

| | | |
|---|---|---|
| 30 | Gew.-% | Anethol |
| 25 | Gew.-% | 1,8-Cineol (Eucalyptol) |
| 45 | Gew.-% | *I*-Menthol |

wurde eine Aromastoff-Komposition für den Einsatz in Mundwassern bzw. Mundwasserkonzentraten hergestellt.

### Beispiel 7.2:

Durch Vermischen von

| | | |
|---|---|---|
| 10 | Gew.-% | Anethol |
| 17,5 | Gew.-% | Eucalyptusöl (70-75% 1,8-Cineol) |
| 17,5 | Gew.-% | 1,8-Cineol (Eucalyptol) |
| 54,4 | Gew.-% | *I*-Menthol |
| 0,6 | Gew.-% | Menthancarbonsäure-N-(4-methoxyphenyl)-amid |

wurde eine Aromastoff-Komposition mit einem Anteil von 0,6 Gew.-% Menthancarbonsäure-N-(4-methoxyphenyl)-amid (WS-12) für den Einsatz in Mundwassern bzw. Mundwasserkonzentraten hergestellt.

Die Aromastoff-Kompositionen gemäß Beispiel 7.1 (für ein Vergleichs-Mundwasser bzw. ein Vergleichs-Mundwasserkonzentrat) und Beispiel 7.2 (für ein hierin beschriebenes Mundwasser bzw. ein hierin beschriebenes Mundwasserkonzentrat) wurden jeweils mit einer Konzentration von 0,15 Gew.-% in jeweils ein gebrauchsfertiges Mundwasser bzw. jeweils mit einer Konzentration von 3 Gew.-% in jeweils ein gebrauchsfertiges Mundwasserkonzentrat eingearbeitet, bezogen auf die Gesamtmasse des resultierenden Mundwassers bzw. des resultierenden Mundwasserkonzentrates.

Die daraus resultierenden Mundwasser bzw. Mundwasserkonzentrate wurden von einem sensorisch geschulten Expertenpanel unter Praxisbedingungen getestet. Die sensorische Bewertung für das Mundwasser/Mundwasserkonzentrat, enthaltend eine Aromastoff-Komposition gemäß Beispiel 7.2, ergab eine starke, angenehm frische Eucalyptus-Note mit einem sehr lang anhaltenden Frischeempfinden, das auch nach Verwendung des Mundwassers/Mundwasserkonzentrates noch über eine Zeit von weit über 30 Minuten anhielt.

Durch den Anteil an 0,6 Gew.-% Menthancarbonsäure-N-(4-methoxyphenyl)-amid (WS-12) in der Aromastoff-Komposition gemäß Beispiel 7.2 konnte der zur Bitterreduktion in Beispiel 7.1 eingesetzte Anteil an Anethol (30 Gew.-%) auf nunmehr 10 Gew.-% reduziert werden. Zudem konnte der linear zugesetzte Menthol-Anteil in der Aromastoff-Komposition von 40 Gew.-% (Beispiel 7.1) auf 54,4 Gew.-% (Beispiel 7.2) erhöht werden. Gleichzeitig konnten 17,5 Gew.-% Eucalyptusöl (zu 70-75% umfassend 1,8-Cineol) zugesetzt werden, ohne dass sich die bitter-scharfe Note des Menthols einstellte.

In der oben beschriebenen Aromastoff-Komposition gemäß Beispiel 7.2 beträgt der Anteil an linear zugesetztem Menthol 54,4 Gew.-%, der dem Gesamtmentholanteil entspricht. Das Gewichtsverhältnis von Menthancarbonsäure-N-(4-methoxyphenyl)-amid (WS-12) zu linear zugesetztem Menthol bzw. zu dem Gesamtmentholgehalt beträgt damit 1 : 91.

Das Gewichtsverhältnis von Menthancarbonsäure-N-(4-methoxyphenyl)-amid (WS-12) zu der Gesamtmenge an weiteren Aromastoffen mit einem log-K_{ow}-Wert im Bereich von 1,5 bis 5 (Anethol, 1,8-Cineol, sowie entsprechender Anteil an 1,8-Cineol im Eucalyptusöl) beträgt 1:68 bis 1: 70, je nach Anteil des 1,8-Cineol im Eucalyptusöl.

### Weitere Anwendungsbeispiele oben beschriebener Aromastoff-Kompositionen in Fertigprodukten:

Die in den Beispielen 1 bis 7 beschriebenen Aromastoff-Kompositionen eignen sich nicht nur für den Einsatz in Zahnpasten, sondern auch für den Einsatz in einer ganzen Reihe weiterer, unterschiedlicher Fertigprodukte. Bei allen nachfolgend aufgeführten Anwendungsbeispielen konnte (in eigenen Tests) ein vorteilhaftes, schnell einsetzendes, gleichzeitig langandauerndes Frischeempfinden wahrgenommen werden, ohne dass dieses Frischeempfinden durch bittere und/oder (unerwünschte) scharfe Noten, die durch die Anwesenheit von Menthol, aber auch durch weitere Substanzen mit einer bitteren und/oder (unerwünschte) scharfen Note entstehen können, negativ beeinträchtigt wurden.

### Beispiel 8: Zahnpasta (,Silica opaque')

| **Bestandteil** | **I (Gew.-%)** | **II (Gew.-%)** |
|---|---|---|
| Entionisiertes Wasser | 26,53 | 26,53 |
| Sorbitol 70% | 45,00 | Ad 100 |
| Solbrol M Na-Salz | 0,15 | 0,15 |
| Trinatriumphosphat | 0,10 | 0,10 |
| Saccharin | 0,20 | 0,20 |
| Natriummonofluorphosphat | 1,12 | 1,12 |
| PEG 1500 | 5,00 | 5,00 |
| Sident 9 (Abrasive Silica) | 10,00 | 10,00 |
| Sident 22 S (Dickende Silica) | 8,00 | 8,00 |
| Natriumcarboxymethylcellulose | 0,90 | 0,90 |
| Titan (IV) oxid | 0,50 | 0,50 |
| Natriumlaurylsulfat (SLS) | 1,50 | 1,50 |
| Pellitorin-Lösung PLM (enthaltend 10% Pellitorin) | - | 0,025 |
| **Aroma Eucalyptus-Menthol-Typ (Beispiel 2.2)** | **1,00** | **1,00** |

### Beispiel 9: Zahnpasta (Calciumcarbonat-Base)

| **Bestandteil** | **I (Gew.-%)** | **II (Gew.-%)** |
|---|---|---|
| Entionisiertes Wasser | 27,50 | Ad 100 |
| Saccharin | 0,20 | 0,20 |
| Solbrol M Natriumsalz | 0,20 | 0,20 |
| Natriummonofluorphosphat | 0,80 | 0,80 |
| Sorbitol 70% | 29,00 | 29,00 |
| Calciumcarbonat | 35,00 | 35,00 |
| Sident 22 S (Verdickende Silica) | 2,50 | 2,50 |
| Natriumcarboxymethylcellulose | 1,30 | 1,30 |
| Titandioxid | 0,50 | 0,50 |
| Natriumlaurylsulfat | 2,00 | 2,00 |
| **Aroma Eucalyptus-Menthol-Typ (Beispiel 2.2)** | **1,00** | **1,00** |
| Pellitorin-Lösung PLM (enthaltend 10% Pellitorin) | - | 0,020 |

### Beispiel 10: Zahnpasta mit Bleichwirkung

| | **I (Gew.-%)** | **II (Gew.-%)** | **III (Gew.-%)** |
|---|---|---|---|
| Polyphosphat (Glass H, (n ≈ 21), Astaris) | 7,00 | 7,00 | 7,00 |
| Calciumperoxid | 1,00 | - | 2,50 |
| Na-percarbonat | - | 11,00 | - |
| Poloxamer 407 | 5,00 | 2,00 | 5,00 |
| Polyethylenglycol | 3,00 | - | 3,00 |
| Sorbitol, 70 %ig in Wasser | - | 22,00 | - |
| Glycerin | 43,80 | 12,50 | 28,60 |
| 1,2-Propylenglycol | 4,00 | - | 2,50 |
| Na-Saccharin | 0,40 | 0,20 | 0,50 |
| Natriumbicarbonat | - | 5,00 | 15,00 |
| Natriumcarbonat | 2,00 | 2,00 | 2,00 |
| Silica | 20,00 | 22,00 | 20,00 |
| Na-Carboxymethylcellulose | 0,60 | 0,55 | 0,30 |
| Natriumlaurylsulfat | 1,00 | 4,00 | 2,00 |
| Xanthan Gum | 0,20 | 0,20 | 0,20 |
| Titandioxid (Anatas) | 0,50 | 0,50 | 0,50 |
| **Aroma Eucalyptus-Menthol-Typ (Beispiel 2.2)** | **1,00** | - | - |
| **Aroma Menthol-Zimt-Typ (Beispiel 4)** | **-** | **1,25** | - |
| **Aroma Zimt-würzig (Beispiel 6.2)** | **-** | **-** | **1,50** |
| Wasser dest. | Ad 100 | Ad 100 | Ad 100 |

### Beispiel 11: Zahnpasten mit Zinn- und Zinksalzen

| | **I (Gew.-%)** | **II (Gew.-%)** | **III (Gew.-%)** |
|---|---|---|---|
| Natriumfluorid NaF | 0,42 | 0,50 | - |
| Zinnfluorid SnF₂ | - | 0,90 | 0,95 |
| Zinnchlorid SnCl₂ | 1,50 | - | 2,00 |
| Zinklactat | 2,00 | 2,00 | - |
| Zinkcarbonat ZnCO₃ | - | 1,00 | 1,50 |
| Na-gluconat | - | 0,67 | 1,50 |
| Poloxamer 407 | 14,50 | - | - |
| Polyethylenglycol | 1,00 | 3,00 | - |
| Sorbitol, 70 %ig in Wasser | - | 38,00 | 37,50 |
| Glycerin | 37,50 | 5,00 | 14,40 |
| 1,2-Propylenglycol | 7,00 | 5,00 | - |
| Na-Saccharin | 0,30 | 0,50 | 0,50 |
| Abrasiv-Silica | 20,00 | 22,50 | 25,00 |
| Natriumhydroxid | - | 0,10 | 0,20 |
| Natriumlaurylsulfat | - | 2,00 | 1,50 |
| Na-polyphosphat | - | - | 4,00 |
| Tetranatriumpyrophosphat | 1,00 | 2,50 | - |
| Farbstoff (1%ig in Wasser) | 0,40 | 0,50 | 0,50 |
| **Aroma Eucalyptus-Menthol-Typ (Beispiel 2.2)** | **0,95** | - | - |
| **Aroma Menthol-Zimt-Typ (Beispiel 4)** | - | **1,20** | - |
| **Aroma Wintergrün (Beispiel 7.2)** | - | - | **1,15** |
| Wasser dest. | Ad 100 | Ad 100 | Ad 100 |

### Beispiel 12: Zahnpasta auf Phosphatbasis

| **Bestandteil** | **Anteil [%]** |
|---|---|
| Entionisiertes Wasser | 36,39 |
| Glycerin | 20,00 |
| Solbrol M (Natriumsalz) | 0,15 |
| Natriummonofluorphosphat | 0,76 |
| Saccharin | 0,20 |
| Dicalciumphosphat-Dihydrat | 36,00 |
| Aerosil® 200 (Silica) | 3,00 |
| Natriumcarboxymethylcellulose | 1,20 |
| Natriumlaurylsulfat (Texapon) | 1,30 |
| **Aroma Krauseminz-Typ (Beispiel 5.2)** | **1,00** |

### Beispiel 13: Zahnpasta (transparente Gelformulierung)

| **Bestandteil** | **I (Gew.-%)** | **II (Gew.-%)** |
|---|---|---|
| Sorbitol 70% | 63,00 | Ad 100 |
| Entionisiertes Wasser | 11,31 | 11,31 |
| Saccharin | 0,20 | 0,20 |
| Natriummonofluorphosphat | 1,14 | 1,14 |
| Solbrol | 0,15 | 0,15 |
| Trinatriumphosphat | 0,10 | 0,10 |
| PEG 1500 (PEG 32) | 5,00 | 5,00 |
| Sident 9 (Abrasive Silica) | 8,00 | 8,00 |
| Sident 22 S (Verdickende Silica) | 8,00 | 8,00 |
| Natriumcarboxymethylcellulose | 0,60 | 0,60 |
| Natriumlaurylsulfat | 1,50 | 1,50 |
| **Aroma Menthol-Zimt-Typ (Beispiel 4)** | **1,00** | **-** |
| **Aroma Wintergrün (Beispiel 7.2)** | **-** | **1,00** |
| Pellitorin-Lösung PLM (enthaltend 10% Pellitorin) | - | 0,025 |

### Beispiel 14: Zahncreme und Mundwasser als 2-in-1 Produkt

| | **I (Gew.-%)** |
|---|---|
| Ethanol, 96%ig | 5,00 |
| Sorbitol, 70 %ig in Wasser | 40,00 |
| Glycerin | 20,00 |
| Saccharin | 0,20 |
| Na-Monofluorphosphat | 0,76 |
| Solbrol M, Na-Salz | 0,15 |
| Abrasivkieselsäure (Sident 9) | 20,00 |
| Verdickungskieselsäure (Sident 22S) | 2,00 |
| Na-Carboxymethylcellu lose | 0,30 |
| Natriumlaurylsulfat | 1,20 |
| Grüner Farbstoff (1%ig in Wasser) | 0,50 |
| **Aroma Eucalyptus-Menthol-Typ (Beispiel 2.2)** | **1,00** |
| Wasser dest. | Ad 100 |

### Beispiel 15: Mundwasserkonzentrat enthaltend eine Aromastoff-Komposition des Typs Wintergrün

| **Bestandteil** | **Anteil [%]** |
|---|---|
| Ethylalkohol 96% | 42,00 |
| Cremophor RH 455 | 5,00 |
| Entionisiertes Wasser | 48,67 |
| Allantoin | 0,20 |
| Natriumsaccharin 450 | 0,10 |
| Colour L-Blue 5000 (1 % in Wasser) | 0,03 |
| **Aroma Wintergrün (Beispiel 7.2)** | **4,00** |

### Beispiel 16: Mundwasser (,ready to use' ohne Alkohol)

| **Bestandteil** | **I (Gew.-%)** | **II (Gew.-%)** |
|---|---|---|
| Cremophor RH 455 | 1,80 | 1,80 |
| Entionisiertes Wasser | 87,57 | Ad 100 |
| Sorbitol 70% | 10,00 | 10,00 |
| Natriumfluorid | 0,18 | 0,18 |
| Natriumsaccharin 450 | 0,10 | 0,10 |
| Solbrol M Natriumsalz | 0,15 | 0,15 |
| **Aroma Eucalyptus-Menthol-Typ (Beispiel 2.2)** | **0,2** | **0,2** |
| Pellitorin-Lösung PLM (enthaltend 10% Pellitorin) | - | 0,0125 |

### Beispiel 17: Mundwasser (,ready to use' mit Alkohol)

| **Bestandteil** | **I (Gew.-%)** | **II (Gew.-%)** | **III (Gew.-%)** |
|---|---|---|---|
| Ethylalkohol 96% | 10,02 | 5,00 | 7,00 |
| Cremophor CO 40 | 1,00 | 1,00 | 1,00 |
| Benzoesäure | 0,10 | 0,12 | 0,10 |
| Entionisiertes Wasser | 83,46 | Ad 100 | Ad 100 |
| Sorbitol 70% | 5,00 | 1,00 | 5,00 |
| Natriumsaccharin 450 | 0,07 | 0,05 | 0,05 |
| L-Blue 5000 (1% in Wasser) | 0,10 | 0,10 | 0,10 |
| Glycerin | - | 8,00 | - |
| 1,2-Propylenglycol | - | 2,00 | 3,00 |
| Cetylpyridiniumchlorid | - | - | 0,07 |
| Wasserstoffperoxid (35% H₂O₂ in Wasser) | - | 3,00 | 4,00 |
| **Aroma Wintergrün (Beispiel 7.2)** | **0,25** | - | - |
| **Aroma Eucalyptus-Menthol-Typ (Beispiel 2.2)** | **-** | **0,25** | **0,05** |

### Beispiel 18: Standard Kaugummi

| **Bestandteil** | **I (Gew.-%)** | **II (Gew.-%)** |
|---|---|---|
| Gum Base (Kaugummibase) | 21,00 | 21,00 |
| Glucosesirup | 16,50 | 16,50 |
| Glycerin | 0,50 | 0,50 |
| Zucker gepulvert | 60,00 | 60,00 |
| **Aroma Eucalyptus-Menthol-Typ (Beispiel 2.2)** | **2,00** | - |
| **Aroma Krauseminz-Typ (Beispiel 5.2)** | **-** | **2,00** |

### Beispiel 19: Zuckerfreier Kaugummi

| **Bestandteil** | **I (Gew.-%)** | **II (Gew.-%)** |
|---|---|---|
| Gum Base (Kaugummibase) | 30,00 | 30,00 |
| Sorbit gepulvert | 40,00 | Ad 100 |
| Isomalt gepulvert | 9,50 | 9,50 |
| Xylitol | 2,00 | 2,00 |
| Mannit D | 3,00 | 3,00 |
| Aspartam | 0,10 | 0,10 |
| Acesulfam K | 0,10 | 0,10 |
| Emulgum™ (Soja-Lecithine mit hohem Gehalt an Phospholipiden) | 0,30 | 0,30 |
| Sorbitol (70% in Wasser) | 13,00 | 13,00 |
| 1,2-Propylenglyol | - | 1,00 |
| Glycerin | 1,00 | - |
| Pellitorin-Lösung PLM (enthaltend 10% Pellitorin) | - | 0,035 |
| **Aroma Menthol-Zimt-Typ (Beispiel 4)** | **1,00** | **1,00** |

### Beispiel 20: Kaugummis (mit Zucker und zuckerfrei)

| | **I (Gew.-%)** | **II (Gew.-%)** |
|---|---|---|
| Gum Base (Kaugummibase) | 21,0 | 30,0 |
| Glycerin | 0,5 | 1,0 |
| **Aroma Krauseminz-Typ (Beispiel 5.2)** | **1,0** | **1,4** |
| Glucosesirup | 16,5 | - |
| Puderzucker | Ad 100 | - |
| Sorbitol (in Pulverform) | - | Ad 100 |
| Palatinit | - | 9,5 |
| Xylitol | - | 2,0 |
| Mannitol | - | 3,0 |
| Aspartam | - | 0,1 |
| Acesulfam K | - | 0,1 |
| Emulgum™ (Emulgator) | - | 0,3 |
| Sorbitol 70%, in Wasser | - | 14,0 |

### Beispiel 21: Zuckerfreie Kaugummis

Die Kaugummibase K1 bestand aus 2,0% Butyl Rubber (Isobuten-Isopren-Copolymer, MW 400000), 6,0% Polyisobuten (MW = 43800), 43,5% Polyvinylacetat (MW = 12000), 31,5% Polyvinylacetat (MW = 47000), 6,75% Triacetin und 10,25% Calciumcarbonat. Die Herstellung der Kaugummibase K1 und der Kaugummis kann analog zu US 5,601,858 erfolgen.

| | **I (Gew.-%)** | **II (Gew.-%)** | **III (Gew.-%)** |
|---|---|---|---|
| Kaugummibase K1 | 26,00 | 27,00 | 26,00 |
| Triacetin | 0,25 | 0,25 | 0,25 |
| Lecithin | 0,50 | 0,50 | 0,50 |
| Sorbitol, kristallin | Ad 100 | Ad 100 | Ad 100 |
| Mannitol | 15,30 | 15,20 | 15,10 |
| Glycerin | 12,10 | 12,00 | 11,80 |
| Saccharin-Na | 0,17 | - | 0,10 |
| Verkapseltes Aspartam | 1,08 | 1,18 | 1,08 |
| Amorphes Silica | 1,00 | 1,00 | 1,00 |
| Baumwollsamenöl | 0,50 | 0,50 | 0,50 |
| Polyoxyethylen-sorbitan-monolaurat (E-432) | 1,00 | 1,00 | 1,00 |
| Verkapseltes I-Carvon (Beladung: 30%) | - | 0,20 | - |
| **Aroma Wintergrün (Beispiel 7.2)** | **1,00** | - | **1,70** |
| **Aroma Eucalyptus-Menthol-Typ (Beispiel 2.2)** | **0,50** | **1,40** | **-** |
| I-Menthyl-I-lactat | - | - | 0,20 |

### Beispiel 22: Zuckerfreie Kaugummis

Die Kaugummibase K2 bestand aus 28,5% Terpenharz, 33,9% Polyvinylacetat (MW = 14000), 16,25% hydriertes Pflanzenöl, 5,5% Mono- und Diglyceride, 0,5% Polyisobuten (MW 75000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis kann analog zu US 6,986,907 erfolgen.

| | **I (Gew.-%)** | **II (Gew.-%)** | **III (Gew.-%)** |
|---|---|---|---|
| Kaugummibase K2 | 25,30 | 27,30 | 26,30 |
| Sorbitol | Ad 100 | Ad 100 | Ad 100 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,68 |
| Menthol, sprühgetrocknet (Beladung: 25%) | 0,50 | - | 0,50 |
| Kirscharoma, sprühgetrocknet (enthält Benzaldehyd) | - | 1,00 | - |
| **Aroma Eucalyptus-Menthol-Typ (Beispiel 2.2);** sprühgetrocknet, Aromagehalt 30% | **1,50** | **1,70** | - |
| **Aroma Menthol-Zimt-Typ (Beispiel 4)** | **1,00** | **-** | **1,50** |

Die Kaugummis der Rezeptur (I) und (II) wurden als Streifen, die der Rezeptur (III) als Kompaktate in Kissenform hergestellt und anschließend mit Xylit dragiert.

### Beispiel 23: Bonbon ('Hardboiled candy'), zuckerfrei

| **Bestandteil** | **I (Gew.-%)** | **II (Gew.-%)** |
|---|---|---|
| Wasser | 2,24 | 2,24 |
| Isomalt | 94,98 | Ad 100 |
| Xylitol | 2,40 | 2,40 |
| Sucralose | 0,03 | 0,03 |
| Acesulfam K | 0,050 | 0,050 |
| Citronensäure | 0,050 | 0,050 |
| Pellitorin-Lösung PLM (enthaltend 10% Pellitorin) | - | 0,0075 |
| **Aroma Eucalyptus-Menthol-Typ (Beispiel 2.2)** | **0,25** | **0,20** |

### Beispiel 24: Bonbons ('Hardboiled candy')

| **Bestandteil** | **I (Gew.-%)** | **II (Gew.-%)** |
|---|---|---|
| Wasser | 2,75 | 2,50 |
| Zucker | 60,1 | Ad 100 |
| Glucosesirup | 36,9 | 36,0 |
| Maltose | - | 2,00 |
| Palmkernöl | - | 0,80 |
| Citronensäure | - | 0,25 |
| Ginseng Extrakt | - | 0,40 |
| Blauer Farbstoff | - | 0,01 |
| **Aroma Krauseminz-Typ (Beispiel 5.2)** | **0,25** | **0,35** |

### Beispiel 25: Instant-Getränkepulver

| | **(Gew.-%)** |
|---|---|
| Zucker (Saccharose) | Ad 100 |
| Citronensäure | 11,58 |
| Trinatriumcitrat | 0,70 |
| Tricalciumphosphat | 0,60 |
| Vitamin C | 0,66 |
| Grindsted® JU 543 Stabilizer System (Danisco) | 0,90 |
| Saccharin | 0,561 |
| Citronenaroma, sprühgetrocknet | - |
| Orangenaroma, sprühgetrocknet | 1,85 |
| **Aroma Menthol-Zimt-Typ (Beispiel 4)** sprühgetrocknet auf Maltodextrin (DE 15-19) und Gummi Arabicum, Aromabeladung 40% | **1,20** |

45 g dieses Instant-Getränkepulvers wurden in 1000 mL unter Rühren gelöst. Das erhaltene Getränk hatte einen erfrischenden, kühlenden Geschmack von Orange, Zimt und Minze.

### Beispiel 26: Halsbonbons mit flüssig-viskoser Kernfüllung (center-filled hard candy) mit Aroma Zimttyp und Aroma Zimttyp Cool

| | **I (Gew.-%)** | **II (Gew.-%)** |
|---|---|---|
| **Mischung A (Hülle)** (80% der Bonbons) | | |
| Zucker (Saccharose) | 58,12 | 49,37 |
| Glucosesirup (Feststoffgehalt 80%) | 41,51 | 49,37 |
| **Aroma Menthol-Zimt-Typ (Beispiel 4)** | **0,17** | **0,25** |
| I-Menthol | 0,10 | - |
| Citronenöl | 0,10 | 0,10 |
| Citronensäure | - | 0,91 |
| Total: | 100 | 100 |

| **Mischung B** (Kern) (20% der Bonbons) | | |
|---|---|---|
| High Fructose Maissirup (Gehalt an festen Zuckern 85%, knapp 15% Wasser) | 84,355 | 84,36 |
| Glycerin | 15,0 | 15,0 |
| Lecithin | 0,02 | 0,02 |
| Zimtöl | - | 0,27 |
| **Aroma Zimt-würzig (Beispiel 6.2)** | **0,28** | **-** |
| Capsaicin | 0,025 | - |
| Piperin | 0,05 | 0,05 |
| Vanillylalkohol-n-butylether | - | 0,10 |
| Roter Farbstoff, als 2,5%ige wässrige Lösung | 0,20 | 0,20 |
| Vanillin | 0,07 | - |
| Total | 100 | 100 |

In Anlehnung an die in US 6,432,441 (dort Beispiel 1) sowie die in US 5,458,894 bzw. US 5,002,791 beschriebenen Verfahren wurden Bonbons mit flüssig-viskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

Der Zusatz scharf schmeckender Substanzen (Capsaicin, Piperin) erfolgt hier zum Erzeugen einer (nicht vollständig maskierten) scharfen Note.

### Beispiel 27: Zum Direktverzehr geeignete Gelatinekapseln

| | **(Gew.-%)** |
|---|---|
| Gelatinehülle: | |
| Glycerin | 2,014 |
| Gelatine 240 Bloom | 7,91 |
| Aspartam | - |
| Sucralose | 0,070 |
| Allura Red (roter Farbstoff) | 0,006 |
| Brillant Blue (blauer Farbstoff) | 0,005 |
| | |

| Kernzusammensetzung: | |
|---|---|
| Pflanzenöltriglyceride (Kokonussölfraktion; coconut oil fraction) | Ad 100 |
| Aroma G* | 7,5 |
| **Aroma Eucalyptus-Menthol-Typ (Beispiel 2.2)** | **18,5** |

Aroma G* hatte dabei folgende Zusammensetzung (Angaben jeweils in Gew.-% bezogen auf das Gesamtgewicht des Aromas): 0,1% Neotam Pulver, 29,3% Pfefferminzöl Mentha arvensis, rektifiziert,, Ad 100% Pfefferminzöl Mentha piperita Typ Willamette, 2,27% Sucralose, 0,7% Gewürznelkenöl (clove bud oil), 2,28% Triacetin, 5,4% Diethyltartrat, 12,1% Pfefferminzöl yakima, 0,7% Ethanol, 3,36% 2-Hydroxyethylmenthylcarbonat, 2,6% 2-Hydroxypropylmenthylcarbonat, 5,77% D-Limonen, 5,67% L-Menthylacetat, 0,4% Vitamin E - acetat.

Die zum Direktverzehr geeigneten Gelatinekapseln wurden gemäß WO 2004/050069 hergestellt und hatten einen Durchmesser von 5 mm; das Gewichtsverhältnis von Kernmaterial zu Hüllmaterial lag bei 90 : 10. Die Kapseln öffneten sich jeweils im Mund innerhalb von weniger als 10 Sekunden und lösten sich vollständig innerhalb von weniger als 50 Sekunden auf.

## Patentansprüche

1. Verwendung einer Aromastoff-Komposition mit den Komponenten
a) Menthol,
b) eine die Bitterkeit des Menthol maskierende Menge an Menthancarbonsäure-N-(4-methoxyphenyl)-amid (WS-12) sowie
c) ein, zwei oder mehr weitere Aromastoffe, wobei der, mehrere oder sämtliche der weiteren Aromastoffe einen log-K0w-Wert im Bereich von 1,5 bis 5 besitzen,
zur Maskierung unangenehmer Geschmackseindrücke von Triclosan, Zinkcitrat, -sulfat, Poly- und Pyrophosphaten, Bicarbonaten, wie z.B. Natriumbicarbonat, Strontium- und Kaliumsalzen, wie z.B. Kaliumcitrat, Kaliumnitrat, Kaliumchlorid, und Strontiumchlorid, Zinnpyrophosphat, -chlorid, Aluminiumlactat, Wasserstoffperoxid, Fluoriden, Vitaminen, Cetylpyridiniumchlorid und Emulgatoren, wie z.B. Natriumlaurylsulfat, Natriumlaurylsarkosinat und Cocamidopropylbetain sowie Süßstoffen wie z.B. Aspartam, Saccharin, Acesulfam-K, Sorbit, Xylit, Cyclamaten wie z.B. Natriumcyclamat, Sucralose, Alitam, Neotam, Thaumatin, von Neohesperidin Dihydrochalcon, Maltit, Lactit oder Kaugummi-Massen (GumBasen) oder deren Mischungen, wobei die unangenehmen Geschmackseindrücke bittere oder metallische Geschmackseindrücke sind.

2. Verwendung nach Anspruch 1, wobei die Süßstoffe ausgewählt sind aus der Gruppe bestehend aus
(i) Aspartam, Saccharin, Acesulfam-K, Sorbit, Xylit, Cyclamaten, wie z.B. Natriumcyclamat, Sucralose, Alitam, Neotam, Thaumatin, Neohesperidin Dihydrochalcon, Maltit und/oder Lactit.

3. Verwendung nach Anspruch 1 oder 2, wobei die Emulgatoren ausgewählt sind aus der Gruppe bestehend aus
(iii) Natriumlaurylsulfat, Natriumlaurylsarkosinat und Cocamidopropylbetain.

4. Verwendung nach einem der vorangehenden Ansprüche, wobei
c) der, mehrere oder sämtliche der weiteren Aromastoffe ausgewählt sind aus der Gruppe bestehend aus Menthon, Isomenthon, Carvon, 1,2-Dihydrocarvon, Anethol, Piperiton, Menthylacetat, Menthylmethylether, 1,8-Cineol, Zimtaldehyd und Methylsalicylat, vorzugsweise aus der Gruppe bestehend aus Menthon, Isomenthon, Carvon und Piperiton.

## Claims

1. Use of a flavouring composition comprising the following components
(a) menthol,
(b) a menthol bitterness masking amount of N-(4-methoxyphenyl)-amide (WS-12) of menthanecarboxylic acid and
c) one, two or more further flavouring substances, wherein the, several or all of the other flavouring substances have a log-KOw value in the range from 1.5 to 5,
for masking unpleasant taste impressions of triclosan, zinc citrate, zinc sulphate, poly- and pyrophosphates, bicarbonates such as e.g. sodium bicarbonate, strontium and potassium salts, such as e.g. potassium citrate, potassium nitrate, potassium chloride and strontium chloride, tin pyrophosphate, tin chloride, aluminium lactate, hydrogen peroxide, fluorides, vitamins, cetylpyridinium chloride and emulsifiers such as e.g. sodium lauryl sulphate, sodium lauryl sarcosinate and cocamidopropyl betaine as well as sweeteners such as e.g. aspartame, saccharin, acesulfame-K, sorbitol, xylitol, cyclamates such as e.g. sodium cyclamate, sucralose, alitame, neotame, thaumatin, from neohesperidin dihydrochalcon, maltitol, lactitol or chewing gum masses (GumBases) or mixtures thereof, the unpleasant taste impressions being bitter or metallic taste impressions.

2. Use according to claim 1, wherein the sweeteners are selected from the group consisting of
(i) aspartame, saccharin, acesulfame-K, sorbitol, xylitol, cyclamates such as sodium cyclamate, sucralose, alitame, neotame, thaumatin, neohesperidine dihydrochalcon, maltitol and/or lactitol.

3. Use according to claim 1 or 2, wherein the emulsifiers are selected from the group consisting of
(iii) sodium lauryl sulfate, sodium lauryl sarcosinate and cocamidopropyl betaine.

4. Use according to any one of the previous claims, wherein
c) the, several or all of the other flavouring substances are selected from the group consisting of menthone, isomenthone, carvone, 1,2-dihydrocarvone, anethole, piperitone, menthyl acetate, menthyl methyl ether, 1,8-cineol, cinnamic aldehyde and methyl salicylate, preferably from the group consisting of menthone, isomenthone, carvone and piperitone.

## Revendications

1. Utilisation d'une composition de substance aromatisante comprenant les composants
a) le menthol,
b) une quantité de N-(4-méthoxyphényl)amide de l'acide carboxylique de men-thane (WS-12), qui masque l'amertume du menthol, ainsi que
c) une, deux autre(s) substance(s) aromatisante(s) ou plus; ladite, plusieurs ou l'ensemble des autres substance(s) aromatisante(s) possédant une valeur log-K0w comprise entre 1,5 et 5,
pour masquer des impressions gustatives désagréables de triclosan, de citrate de zinc, de sulfate de zinc, de poly- et pyrophosphates, de bicarbonates tel que, par exemple, le bicarbonate de sodium, de sels de strontium et de potassium tel que, par exemple, le citrate de potassium, le nitrate de potassium, le chlorure de potassium et le chlorure de strontium, de pyrophosphate d'étain, de chlorure d'étain, de lactate d'aluminium, de peroxyde d'hydrogène, de fluorures, de vitamines, de chlorure de cétylpyridinium et d'émulsifiants, tel que, par exemple, le laurylsulfate de sodium, le lauroyl sarcosinate de sodium et la bétaïne de cocamidopropyle, ainsi que d'édulcorants tels que, par exemple, l'aspartame, la saccharine, l'acésulfame-K, le sorbitol, le xylitol, les cyclamates tels que, par exemple, le cyclamate de sodium, le sucralose, l'alitame, le néotame, la thaumatine, de néohespéridine dihydrochalcone, de maltitol, de lactitol ou de bases de gomme (GumBases) ou de leurs mélanges, les impressions gustatives désagréables étant des impressions gustatives amères ou métalliques.

2. Utilisation selon la revendication 1, dans laquelle les édulcorants sont choisis dans le groupe constitué par
(i) l'aspartame, la saccharine, l'acésulfame-K, le sorbitol, le xylitol, les cyclamates tels que, par exemple, le cyclamate de sodium, le sucralose, l'alitame, le néotame, la thaumatine, la néohespéridine dihydrochalcone, le maltitol et/ou le lactitol.

3. Utilisation selon la revendication 1 ou 2, dans laquelle les édulcorants sont choisis dans le groupe constitué par
(iii) le laurylsulfate de sodium, le lauroyl sarcosinate de sodium et la bétaïne de cocamidopropyle.

4. Utilisation selon l'une quelconque des revendications précédentes, dans laquelle
c) ladite, plusieurs ou toutes les autre(s) substance(s) aromatisante(s) sont choisies dans le groupe constitué par la menthone, l'isomenthone, la carvone, la 1,2-dihydrocarvone, l'anéthole, la pipéritone, l'acétate de menthyle, l'éther méthylique de menthyle, le 1,8-cinéole, l'aldéhyde cinnamique et le salicylate de méthyle, de préférence dans le groupe constitué par la menthone, l'isomenthone, la carvone et la pipéritone.
